# EUROPEAN PATENT SPECIFICATION

(11) **EP 2 411 812 B1**
(45) Date of publication and mention of the grant of the patent: **02.10.2013**
(21) Application number: 10710609.8
(22) Date of filing: 24.03.2010
(51) Int. Cl.: G01N 33/564, G01N 33/68

(54) **METABOLIC PROFILES**
STOFFWECHSELPROFILE
PROFILS MÉTABOLIQUES

(30) Priority: 24.03.2009 GB 0905090; 24.03.2009 GB 0905096; 24.03.2009 GB 0905098
(43) Date of publication of application: 01.02.2012
(73) Proprietor: AnaMar AB, 411 36 Goteborg (SE)
(72) Inventor: LUNDSTEDT, Erik, Torbjörn, S-756 55 Uppsala (SE); TRYGG, Nils, Johan, S-904 35 Umea (SE); GABRIELSSON, Jon, Robert, S-903 39 Umea (SE); EKSTRÖM, Gunilla, S-227 38 Lund (SE)
(74) Representative: Webber, Philip Michael
(86) International application number: PCT/GB2010/000557
(87) International publication number: WO 2010/109192

(56) References cited:
- WO-A2-2008/035204
- HERNANZ ANGEL ET AL: "Increased plasma levels of homocysteine and other thiol compounds in rheumatoid arthritis women" CLINICAL BIOCHEMISTRY, vol. 32, no. 1, February 1999 (1999-02), pages 65-70, XP002588908 ISSN: 0009-9120
- TRANG L E ET AL: "PLASMA AMINO-ACIDS IN RHEUMATOID ARTHRITIS" SCANDINAVIAN JOURNAL OF RHEUMATOLOGY, ALMQVIST & WIKSELL PERIODICAL CO., STOCKHOLM, SE, vol. 14, no. 4, 1 January 1985 (1985-01-01), pages 393-402, XP009135225 ISSN: 0300-9742
- KALLIOMÄKI J L ET AL: "Oral tyrosine tolerance test in rheumatoid arthritis." ANNALS OF THE RHEUMATIC DISEASES SEP 1966 LNKD- PUBMED:5915591, vol. 25, no. 5, September 1966 (1966-09), pages 469-471, XP002588909 ISSN: 0003-4967

## Description

The invention relates to an *in vitro* method for the detection of disturbances in the metabolic profiles of endogenous metabolites in subjects which are caused by rheumatoid arthritis. Such disturbances can be normalised by treatment of the subject with specified compounds, particularly N-(2-chloro-3,4-dimethoxybenzylideneamino) guanidine.

### Rheumatoid arthritis

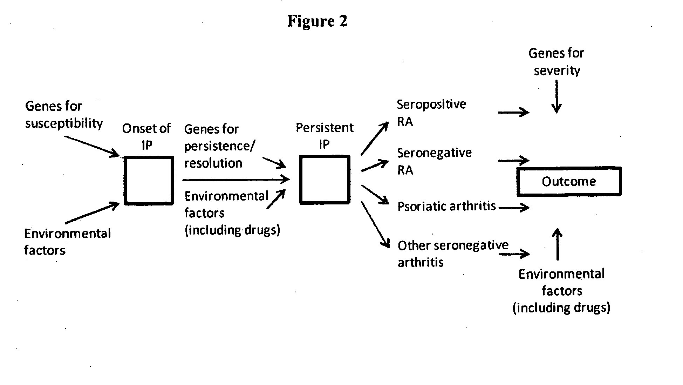

RA is a chronic, inflammatory autoimmune disease (AD) that causes the immune system to attack the joints, which results in a disabling and painful condition that can lead to substantial loss of mobility due to joint destruction and the associated pain (Scott, D. L. et al., Best Pract. Res. Clin. Rheumatol. 21: 943-967 (2007)). The aetiology behind RA is largely unknown (Jefferies, W. M., Medical Hypotheses. 51: 111-114 (1998); Krishnan, E., Joint Bone Spine. 70: 496-502 (2003); Klareskog, L. et al., Arthritis and Rheumatism. 54: 38-46 (2006)). It is likely that the first symptoms are undifferentiated inflammatory arthritis (UIA) rather than RA, and that the UIA may evolve into RA (Dixon, W. G. et al., Best Pract. Res. Clin. Rheumatol. 19: 37-53 (2005)). The evolution of inflammatory arthritis can be divided into four stages (Figure 2).

Early diagnosis is crucial for more effective treatment to prevent irreversible joint damage (Emery, P., Br. J. Rheumatol. 33: 765-768 (1994); van Aken, J. et al., Ann. Rheum. Dis. 63: 274-279 (2004); Emery, P., Br. Med. J. 332: 152-155 (2006)). Detection of patients who go on to develop chronic joint inflammation would aid in targeting those in need of treatment and avoid unnecessary treatment of those less likely to develop the disease (Emery, P. et al., Rheumatol. Int. 27: 793-806 (2007); Emery, P. et al., Rheumatology. 47: 392-398 (2008)). Hence there is a need for methods to diagnose the autoimmune response which is UIA before it evolves into RA (Firestein, G. S., Arthritis Res. Ther. 7: 157-159 (2005)). Trang et al (Scandinavian Journal of Rheumatology 14(4) : 393-402 (1985)) have shown that patients with RA have altered plasma levels of amino acids. Similarly, Hernanz et al. (Clinical Biochemistry 32(1) : 65-70 (1999)) demonstrated increased plasma levels of homocysteine and other thiol compounds in women with RA. Measuring concentrations of endogenous metabolites has the potential to not only diagnose the disease but also to provide new clues to the mechanisms involved in pathogenesis. In the examples we show that it is possible to separate subjects suffering from RA from subjects with related diseases and healthy volunteers by analyzing data generated by GC-MS (gas chromatography - mass spectrometry) of blood plasma, see Table 3. This is crucial for development of drugs that act to delay or even prevent the onset of RA.

**Table 3: Metabolites from human RA**

| **Metabolite** | **Direction in RA** | **Significance, p** |
|---|---|---|
| Isoleucine | ↓ | <0.001 |
| Glycine | ↓ | 0.03 |
| Succinate | ↓ | <0.001 |
| Glyceric acid | ↓ | <0.001 |
| Serine | ↓ | <0.001 |
| Threonine | ↓ | <0.001 |
| Malic acid | ↓ | <0.001 |
| Methionine | ↓ | 0.002 |
| Aspartate | ↓ | <0.001 |
| Pyroglutamate | ↓ | <0.001 |
| 4-Hydroxyproline | ↓ | <0.001 |
| Threonic acid | ↓ | 0.005 |
| Glutamate | ↓ | <0.001 |
| Phenylalanine | ↑ | 0.62 |
| Aspargine | ↓ | <0.001 |
| Glutamine | ↑ | 0.64 |
| Ornithine | ↓ | <0.001 |
| Glucose | ↑ | <0.001 |
| Lysine | ↓ | <0.001 |
| Tyrosine | ↓ | 0.001 |
| Tryptophan | ↓ | <0.001 |
| alfa-tocopherol | ↑ | <0.001 |

A set of endogenous metabolites has now been found that identifies subjects at risk of developing an AD. A subject's profile of these endogenous metabolites may therefore aid in the prognosis, detection and diagnosis of AD, which can be carried out before any clinical symptoms occur, and this enables preventative treatment or prophylaxis to be started early so as to minimise any longer-term tissue damage. The profile of endogenous metabolites may also be used as a tool for screening and identification of drugs and new chemical entities (NCEs) which can act to restore normal levels of these endogenous metabolites, thereby preventing or delaying the onset of the AD and thus being efficacious in the treatment of AD.

We have identified a compound which in our tests has shown ability restore endogenous metabolite levels and which will therefore be of benefit in preventing, delaying or reducing the onset of diseases or disorders, particularly those believed of autoimmune origin, in many of its forms.

The terms "biomarker" and "endogenous metabolite", and the plurals thereof, are used herein interchangeably.

In one embodiment, the invention provides an *in vitro* method for the detection of a disturbance in the metabolic profile of endogenous metabolites in a subject caused by rheumatoid arthritis (RA), the method comprising:
(i) measuring the levels of N endogenous metabolites in a biological sample obtained from the subject, wherein N is 11, in order to produce a metabolic profile of the N endogenous metabolites in that subject;
(ii) comparing the measured levels of the N endogenous metabolites with the corresponding levels of the endogenous metabolites in a biological sample obtained from a control;
wherein the N endogenous metabolites are selected from the group consisting of:
- EM1: Phenylalanine
- EM2: Tyrosine
- EM3: Isoleucine
- EM8: Glycine
- EM9: Glutamine
- EM10: Methionine
- EM14: Lysine
- EM15: Asparagine
- EM16: Serine
- EM17: Tryptophan
- EM18: Threonine
wherein the disturbance in the metabolic profile is one wherein there is a decrease in the level of each of the N measured endogenous metabolites in the biological sample obtained from the subject compared to the corresponding levels of endogenous metabolites in the biological sample obtained from the control, and wherein the disturbance in the metabolic profile is due to RA in the subject,

The invention also provides an *in vitro* method for the detection of a disturbance in the metabolic profile of endogenous metabolites in a subject caused by rheumatoid arthritis (RA), the method comprising:
(i) measuring the levels of N endogenous metabolites in a biological sample obtained from the subject, wherein N is 11, in order to produce a metabolic profile of the N endogenous metabolites in that subject;
(ii) comparing the measured levels of the N endogenous metabolites with the corresponding levels of the endogenous metabolites in a biological sample previously obtained from the subject;
wherein the N endogenous metabolites are selected from the group consisting of:
- EM1: Phenylalanine
- EM2: Tyrosine
- EM3: Isoleucine
- EM8: Glycine
- EM9: Glutamine
- EM10: Methionine
- EM14: Lysine
- EM15: Asparagine
- EM16: Serine
- EM17: Tryptophan
- EM18: Threonine
wherein the disturbance in the metabolic profile is one wherein there is a decreases in the level of each of the N measured endogenous metabolites in the biological sample obtained from the subject compared to the corresponding levels of endogenous metabolites in the biological sample previously obtained from the subject; and wherein the disturbance in the metabolic profile is due to RA in the subject.

Also disclosed is a method of diagnosing or detecting RA in a subject, the method comprising:
(i) measuring the levels of N biomarkers in a biological sample obtained from the subject, wherein N is 2-11;
(ii) comparing the measured levels of the N biomarkers with the corresponding levels of the biomarkers in a biological sample obtained from a control;
wherein the N biomarkers are selected from the group consisting of:
- EM1: Phenylalanine
- EM2: Tyrosine
- EM3: Isoleucine
- EM8: Glycine
- EM9: Glutamine
- EM10: Methionine
- EM14: Lysine
- EM15: Asparagine
- EM16: Serine
- EM17: Tryptophan
- EM18: Threonine
wherein a decrease in the level of each of the N measured biomarkers in the biological sample obtained from the subject compared to the corresponding levels of biomarkers in the biological sample obtained from the control is indicative of the presence of RA in the subject.

Also disclosed is a method of monitoring RA progression in a subject, the method comprising:
(i) measuring the levels of N biomarkers in a biological sample obtained from the subject, wherein N is 2-11;
(ii) comparing the measured levels of the N biomarkers with the corresponding levels of the biomarkers in a biological sample previously obtained from the subject;
wherein the N biomarkers are selected from the group consisting of:
- EM1: Phenylalanine
- EM2: Tyrosine
- EM3: Isoleucine
- EM8: Glycine
- EM9: Glutamine
- EM10: Methionine
- EM14: Lysine
- EM15: Asparagine
- EM16: Serine
- EM17: Tryptophan
- EM18: Threonine
wherein differences in the levels of the N measured biomarkers in the biological sample obtained from the subject compared to the corresponding levels of biomarkers in the biological sample previously obtained from the subject is indicative of a change in the RA prognosis in the subject.

In particular, an increase in the level of each of the N measured biomarkers is indicative of an improvement in the RA prognosis in the subject.

Furthermore, a decrease in the level of each of the N measured biomarkers is indicative of a decline in the RA prognosis in the subject.

Also disclosed is a method for monitoring the rate of decline or rate of improvement of RA in a subject, comprising a method for monitoring RA progression as described herein, comprising the additional step of dividing the increase or decrease of biomarker levels by the time interval between the taking of the first (i.e. previous) and second samples from the subject.

Also disclosed is a method of measuring the effectiveness of a medicament which has been administered to a subject to treat RA in that subject, the method comprising:
(i) measuring the levels of N biomarkers in a biological sample obtained from the treated subj ect, wherein N is 2-11;
(ii) comparing the measured levels of the N biomarkers with the corresponding levels of the biomarkers in a biological sample previously obtained from the subject;
wherein the N biomarkers are selected from the group consisting of:
- EM1: Phenylalanine
- EM2: Tyrosine
- EM3: Isoleucine
- EM8: Glycine
- EM9: Glutamine
- EM10: Methionine
- EM14: Lysine
- EM15: Asparagine
- EM16: Serine
- EM17: Tryptophan
- EM18: Threonine
wherein differences in the levels of the N measured biomarkers in the biological sample obtained from the treated subject compared to the corresponding levels of biomarkers in the biological sample previously obtained from the subject provide an indication of the efficacy of the medicament in that subject.

In particular, an increase in the level of each of the N measured biomarkers is indicative of the medicament being efficacious.

Furthermore, a decrease in the level of each of the N measured biomarkers is indicative of a lack of efficacy of the medicament.

Also disclosed is a method of measuring the effectiveness of a medicament which has been administered to a subject to treat RA in that subject, the method comprising:
(i) measuring the levels of N biomarkers in a biological sample obtained from the treated subject, wherein N is 2-11;
(ii) comparing the measured levels of the N biomarkers with the corresponding levels of the biomarkers in a biological sample obtained from a control;
wherein the N biomarkers are selected from the group consisting of:
- EM1: Phenylalanine
- EM2: Tyrosine
- EM3: Isoleucine
- EM8: Glycine
- EM9: Glutamine
- EM10: Methionine
- EM14: Lysine
- EM15: Asparagine
- EM16: Serine
- EM17: Tryptophan
- EM18: Threonine
wherein differences in the levels of the N measured biomarkers in the biological sample obtained from the treated subject compared to the corresponding levels of biomarkers in the biological sample obtained from the control provide an indication of the efficacy of the medicament in that subject.

In particular, an increase in the level of each of the N measured biomarkers is indicative of the medicament being efficacious.

Furthermore, a decrease in the level of each of the N measured biomarkers is indicative of a lack of efficacy of the medicament.

Also disclosed is a method for monitoring the effectiveness of a medicament which has been administered to a subject to treat RA as described above, which comprises the additional step of administering the medicament to the subject prior to step (i) or in the interval between the taking of samples.

In the context of the above methods for monitoring the effectiveness of a medicament, in some embodiments, the medicament has been administered to the subject before the two biological samples have been obtained. In other embodiments, the medicament has been administered to the subject in the interval between the taking of the two samples.

In some embodiments, the term "method of diagnosing or detecting RA" means a method for detecting probable RA in a subject or a method of determining an increased likelihood of RA in a subject.

Also disclosed is a method of preventing, delaying or reducing the onset of, or treating earlier than hitherto possible, a disturbed metabolic profile in a subject, which comprises:
a) detecting a disturbed metabolic profile caused by rheumatoid arthritis, by a method comprising:
   (i) measuring the levels ofN endogenous metabolites in a biological sample obtained from the subject, wherein N is 2-11, in order to produce a metabolic profile of the N endogenous metabolites in that subject;
   (ii) comparing the measured levels of the N endogenous metabolites with the corresponding levels of the endogenous metabolites in a biological sample obtained from a control;
   wherein the N endogenous metabolites are selected from the group consisting of:
   - EM1: Phenylalanine
   - EM2: Tyrosine
   - EM3: Isoleucine
   - EM8: Glycine
   - EM9: Glutamine
   - EM10: Methionine
   - EM14: Lysine
   - EM15: Asparagine
   - EM16: Serine
   - EM17: Tryptophan
   - EM18: Threonine
wherein the disturbance in the metabolic profile is one wherein there is independently a decrease or increase in the level of each of the N measured endogenous metabolites in the biological sample obtained from the subject compared to the corresponding levels of endogenous metabolites in the biological sample obtained from the control;
and wherein the disturbance in the metabolic profile is due to rheumatoid arthritis; and
(b1) prescribing or supplying to the subject or recommending treatment of the subject with an amount of N-(2-chloro-3,4-dimethoxybenzylideneamino) guanidine or an aminoguanidine, either as the free base or in salt form, effective to prevent, delay, reduce or treat the onset of rheumatoid arthritis, or
(b2) administering to said subject an amount of N-(2-chloro-3,4-dimethoxybenzylideneamino) guanidine or an aminoguanidine, either as the free base or in salt form, effective to prevent, delay, reduce or treat the onset of rheumatoid arthritis.

Also disclosed is a method of preventing, delaying or reducing the onset of, or treating earlier than hitherto possible, a disturbed metabolic profile in a subject, which comprises:
(a) detecting a disturbed metabolic profile caused by rheumatoid arthritis, by a method comprising:
   (i) measuring the levels of N endogenous metabolites in a biological sample obtained from the subject, wherein N is 2-11, in order to produce a metabolic profile of the N endogenous metabolites in that subject;
   (ii) comparing the measured levels of the N endogenous metabolites with the corresponding levels of the endogenous metabolites in a biological sample previously obtained from the subject;
   wherein the N endogenous metabolites are selected from the group consisting of:
   - EM1: Phenylalanine
   - EM2: Tyrosine
   - EM3: Isoleucine
   - EM8: Glycine
   - EM9: Glutamine
   - EM10: Methionine
   - EM14: Lysine
   - EM15: Asparagine
   - EM16: Serine
   - EM17: Tryptophan
   - EM18: Threonine
wherein the disturbance in the metabolic profile is one wherein there is independently a decrease or increase in the level of each of the N measured endogenous metabolites in the biological sample obtained from the subject compared to the corresponding levels of endogenous metabolites in the biological sample previously obtained from the subject;
and wherein the disturbance in the metabolic profile is due to rheumatoid arthritis;
and
(b1) prescribing or supplying to the subject or recommending treatment of the subject with an amount of N-(2-chloro-3,4-dimethoxybenzylideneamino) guanidine or an aminoguanidine, either as the free base or in salt form, effective to prevent, delay, reduce or treat the onset of rheumatoid arthritis, or
(b2) administering to said subject an amount of N-(2-chloro-3,4-dimethoxybenzylideneamino) guanidine or an aminoguanidine, either as the free base or in salt form, effective to prevent, delay, reduce or treat the onset of rheumatoid arthritis.

In some preferred embodiments N is 3-11, 4-11, 5-11, 6-11, 7-11, 8-11, 9-11 or 10-11. In other preferred embodiments, N is 2-5, 3-5 or 4-5. In yet other preferred embodiments, N is 2, 3, 4, 5, 6, 7, 8, 9, 10 or 11. In still other preferred embodiments, the endogenous metabolites are selected from EMs 1-3 and/or EMs 8-10 and/or EMs 14-18; or the endogenous metabolites are selected from EM2, EM3, EM8 and EM10.

The term "measuring" includes assaying, quantifying, imaging or otherwise establishing the presence or absence of the target endogenous metabolite profile, subunits thereof, or combinations of reagent bound targets, and the like, or assaying for, imaging, ascertaining, establishing, or otherwise determining one or more factual characteristics of a disease or disorder as defined herein.

The levels of the N individual endogenous metabolites in the biological samples may be measured using the same or different techniques.

The endogenous metabolite profile may be measured using any suitable means. For example, the endogenous metabolite profile may be measured using chromatographic and/or spectroscopic methods or a reagent that detects or binds to the endogenous metabolites in the profile, preferably using individual antibodies specifically reactive with the individual endogenous metabolites of the profile or a part thereof.

A set of endogenous metabolites has now been found that identifies subjects at risk of developing certain diseases or disorders. A subject's profile of these endogenous metabolites may therefore aid in the prognosis, detection and diagnosis of those diseases or disorders. The profile of endogenous metabolites may also be used as a tool for screening and identification of drugs and new chemical entities (NCEs) which act to restore normal levels of these endogenous metabolites, thereby preventing or delaying the onset of these diseases or disorders and thus being efficacious in the treatment of those diseases or disorders.

The compound N-(2-chloro-3,4-dimethoxybenzylideneamino) guanidine, used herein either as the free base or in salt form, e.g. as the acetate, m.pt. 198-200°C, is a known compound. Its preparation and formulations suitable for containing it have been described in, e.g., WO 02/11715.

The invention further provides the compound N-(2-chloro-3,4-dimethoxybenzylideneamino) guanidine, either as the free base or in salt form, for use as claimed in claim 12.

Examples of suitable chromatographic and spectroscopic methods include Fourier Transform Infra-Red (FT-IR) spectroscopy, nuclear magnetic resonance (NMR) spectroscopy, high-performance liquid chromatography (HPLC), liquid chromatography-mass spectrometry (LC-MS) and gas chromatography-mass spectrometry (GC-MS).

The endogenous metabolite profile may be measured by a chromatography-based technique. The endogenous metabolite compounds can be detected as they are or they can be chemically transformed (derivatized) before detection. Methods of detection can be, but are not limited to, light absorbance of the sample, preferably a UV-detector, or by measuring the mass / charge ratio by a mass spectrometer (e.g. Fonteh, A. N. et al., Amino Acids. 32: 203-212 (2007)).

Other methods for measuring endogenous metabolites include spectroscopic techniques used in conjunction with chemometric methods, e.g. principal component analysis (PCA), partial least squares projections to latent structures (PLS), orthogonal PLS (OPLS), PLS discriminant analysis (PLS-DA) and orthogonal PLS-DA (OPLS-DA).

Other methods for measuring endogenous metabolites include taking a multi-wavelength spectroscopy measurement, typically a transmission spectrum of the sample in e.g. the near-infrared range of the electromagnetic spectrum and comparing the spectrum with a standard sample spectrum from control subject. From a comparison based on chemometric methods it is then determined whether the sample indicates a disease or disorder as defined herein..

In a preferred embodiment of the invention, the levels of one or more of the N endogenous metabolites are independently measured using reagents which individually bind to the individual endogenous metabolites, and which are then detected.

Preferably, the levels of one or more of the N endogenous metabolites are measured using individual antibodies which are specific for the N endogenous metabolites.

The antibodies may be directly or indirectly labelled using a detectable label. Examples of detectable labels include enzymes. Preferably, the substrate for the enzyme is selected so that the substrate, or a reaction product of the enzyme and substrate, forms a fluorescent complex; and the level of the endogenous metabolite is measured by measuring the level of the fluorescent complex.

The antibodies specific for the endogenous metabolite profile used in the methods of the invention may be obtained from scientific or commercial sources. Alternatively, isolated native constituents of the endogenous metabolite profile or recombinant constituents of the endogenous metabolite profile may be utilized to prepare antibodies, monoclonal or polyclonal antibodies, and immunologically active fragments (e.g. a Fab or (Fab)₂ fragment), an antibody heavy chain, an antibody light chain, humanized antibodies, a genetically engineered single chain Fv molecule (Ladner et al, U.S. Pat. No. 4,946,778). Antibodies including monoclonal and polyclonal antibodies, fragments and chimeras, may be prepared using methods known to those skilled in the art.

Antibodies specifically reactive with the endogenous metabolite profile, or derivatives thereof, may be used to detect the endogenous metabolite profile in various biological samples, for example they may be used in any known immunoassays which rely on the binding interaction between an antigenic determinant of a protein and the antibodies. Examples of such assays are radioimmunoassays, enzyme immunoassays (e.g. ELISA), immunofluorescence, immunoprecipitation, latex agglutination, hemagglutination, and histochemical tests.

The sample, or antibodies specific for the endogenous metabolite profile, may be immobilized. Examples of suitable carriers are agarose, cellulose, dextran, Sephadex, Sepharose, liposomes, carboxymethyl cellulose polystyrene, filter paper, ion-exchange resin, plastic film, plastic tube, glass beads, polyamine-methyl vinyl-ether-maleic acid copolymer, amino acid copolymer, ethylene-maleic acid copolymer, nylon, silk, etc. The carrier may be in the shape of, for example, a tube, test plate, well, beads, disc, sphere etc. The immobilized antibody may be prepared by reacting the material with a suitable insoluble carrier using known chemical or physical methods, for example, cyanogen bromide coupling.

In accordance with an embodiment, determining the endogenous metabolite profile in a sample is achieved by measuring the endogenous metabolite profile immunoassay. A variety of immunoassay methods can be used to measure the endogenous metabolite profile. In general, an immunoassay method for the detection of the endogenous metabolite profile may be competitive or non-competitive. Competitive methods typically employ immobilized or immobilizable antibodies to the endogenous metabolite profile and labelled forms of the endogenous metabolite profile. Sample of the endogenous metabolite profile and labelled constituents of the endogenous metabolite profile compete for binding to antibodies specific for the constituents of the endogenous metabolite profile. After separation of the resulting labelled endogenous metabolite profile constituents that has become bound to antibodies specific for the endogenous metabolite profile (bound fraction) from that which has remained unbound (unbound fraction), the amount of the label in either bound or unbound fraction is measured and may be correlated with the amount of the endogenous metabolite profile in the test sample in any conventional manner, e.g., by comparison to a standard curve.

The above-described immunoassay methods and formats are intended to be exemplary and are not limiting since, in general, it will be understood that any immunoassay method or format can be used in the present invention.

The terms "sample", "biological sample", and the like mean a material known to or suspected of containing or expressing the endogenous metabolites in the profile. The test sample can be used directly as obtained from the source or following a pre-treatment to modify the character of the sample. The sample can be derived from any biological source, such as tissues or extracts, including cells, and physiological fluids, such as, for example, whole blood, plasma, serum; saliva, ocular lens fluid, cerebrospinal fluid, sweat, urine, milk, ascites fluid, synovial fluid, peritoneal fluid and the like. The sample can be obtained from animals, preferably mammals, most preferably humans. The sample can be treated prior to use, such as preparing plasma from blood, diluting viscous fluids, and the like. Methods of treatment can involve filtration, distillation, extraction, concentration, inactivation of interfering components, the addition of reagents, and the like. In a preferred embodiment, the biological sample is a biological fluid, more preferably blood or synovial fluid.

The term "subject" refers to a warm-blooded animal such as a mammal which is afflicted with, or suspected to be afflicted with a disease or disorder as defined herein. Preferably, the subject is a human.

As used herein, the term "control" relates to an individual or group of individuals of the same species as the subject being tested. For example, if the subject is a human, the control will also be a human.

The "control" will generally be a group of one or more individuals who show no signs of exhibiting symptoms of a disease or disorder as defined herein. In particular, the controls may be individuals or groups of individuals who are considerably younger than the subject to be tested (e.g. individuals under the age of 30, 40, 50 or 60) or the controls may be unaffected genetic relations who may be age-matched.

In some embodiments of the invention, the control or control group is of the same age or approximately the same age as the subject.

The endogenous metabolite levels in the control may, for example, be available from published charts, computer databases, look-up tables, etc. In other embodiments, the term encompasses a level which has previously been determined. Thus the method of the invention is not limited to methods which comprise the step of physically testing the level of endogenous metabolite obtained from a control.

Levels for control samples from healthy subjects may be established by prospective and/or retrospective statistical studies. Healthy subjects who have no clinically evident disease or abnormalities may be selected for statistical studies. Diagnosis may be made by a finding of statistically different levels of endogenous metabolite profile compared to a control sample or previous levels quantified for the same subject.

Preferably, the changes in the levels of the endogenous metabolites (between the subject and the control samples or between the samples taken at different time intervals from the subject) are significant changes.

In some embodiments of the present invention, a significant increase is one where the level of measured endogenous metabolite in the biological sample obtained from the subject is more than a 10%, 20%, 30%, 40%, 50%, 60%, 70%, 80%, 90% or 100% increase compared to the corresponding level in the biological sample obtained from a control. In other embodiments of the invention, a significant increase means that the increase is significant using the criteria p<0.05, 2-tailed test.

In other embodiments of the present invention, a significant decrease is one where the level of measured endogenous metabolite in the biological sample obtained from the subject is more than a 10%, 20%, 30%, 40%, 50%, 60%, 70%, 80%, 90% or 100% decrease compared to the corresponding level in the biological sample obtained from a control. In other embodiments of the invention, a significant decrease means that the decrease is significant using the criteria p<0.05, 2-tailed lest.

The methods of the invention will be carried out *in vitro*. In particular, the term "biological sample obtained from" a subject or control is intended to indicate that the methods are not carried out on the human or animal body. In particular, the term "obtained from" may comprise receiving a sample from an agent acting on behalf of the subject, e.g. receiving a sample from a doctor, nurse, hospital, medical centre, etc., either directly or indirectly, e.g. via a courier or postal service.

In the methods of the invention where the levels of endogenous metabolites in,a biological sample are compared to the corresponding levels in a biological sample previously obtained from a subject, the time interval between the taking of samples may be any appropriate period, e.g. 1-60 months, 1-36 months, 1-12 months or 1-6 months.

The methods of the invention may be carried out using a diagnostic kit for quantifying the endogenous metabolite profile in a biological sample.

Also disclosed is a kit for use in a method as defined herein, comprising reagents for detecting the presence ofN endogenous metabolites selected from the group consisting of:
- EM1: Phenylalanine
- EM2: Tyrosine
- EM3: Isoleucine
- EM8: Glycine
- EM9: Glutamine
- EM10: Methionine
- EM14: Lysine
- EM15: Asparagine
- EM16: Serine
- EM17: Tryptophan
- EM18: Threonine
wherein N is 2-11, optionally together with instructions for use. Preferably, N is 3-11, 4-11, 5-11, 6-11, 7-11, 8-11, 9-11, 10-11 or 11.

Also disclosed is the use of a kit of the invention for the diagnosis or detection of a disease or disorder as defined herein.

By way of example, the kit may contain antibodies specific for the N endogenous metabolites, antibodies against the antibodies labelled with an enzyme; and a substrate for the enzyme. The kit may also contain one or more of microtiter plate wells, standards, assay diluent, wash buffer, adhesive plate covers, and/or instructions for carrying out a method of the invention using the kit.

The kit may contain a solution for extraction of N endogenous metabolites, a suitable system of eluents, suitable internal or external standards and possibly a suitable chromatographic column and possibly reagents for derivatization of the endogenous metabolite compounds. One example of a commercially available technique for this quantification is the UPLC Amino Acid Analysis Solution from Waters Corporation (Waters, Corporation).

### BRIEF DESCRIPTION OF THE FIGURES

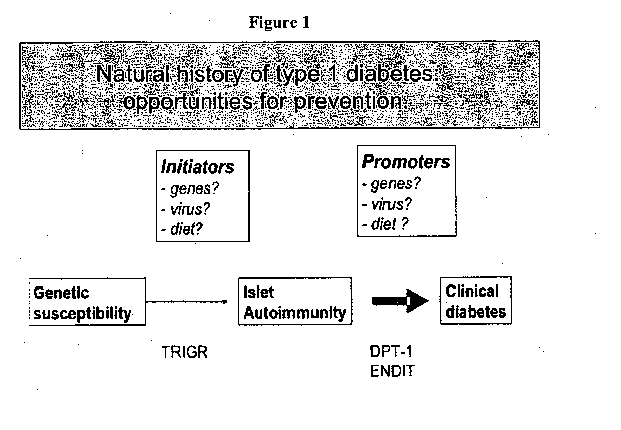

**Figure 1****.** Natural history of type 1 diabetes. TRIGR is a current prevention trial aiming at preventing the development of islet autoimmunity. DPT-1 and ENDIT are past clinical trials in islet autoantibody positive subjects with predictable risk for type 1 diabetes. Metabolomics analyses of serum may define changes due to initiators before the appearance of islet autoimmunity or to promoters inducing hyperglycemia i.e. the onset of clinical diabetes.
**Figure 2****.** Four stages of evolution of inflammatory arthritis, adapted from (Dixon, W. G. et al., Best Pract. Res. Clin. Rheumatol. 19: 37-53 (2005)). During the first stage leading up to the onset of symptoms, the combination of genetic and environmental factors triggers an autoimmune response and the development of joint inflammation in the patient. This phase may share many common features for all ADs. In the second stage persistence of the inflammatory polyarthritis (IP) is determined. Again, for reasons largely unknown, for some patients the joint inflammation is temporary and some patients develop a chronic inflammation. In the third and fourth phases the type of arthritis and severity of the disease is determined. A large proportion of patients who develop an acute IP will make a complete recovery within days or a few weeks, often without even consulting the primary care unit.

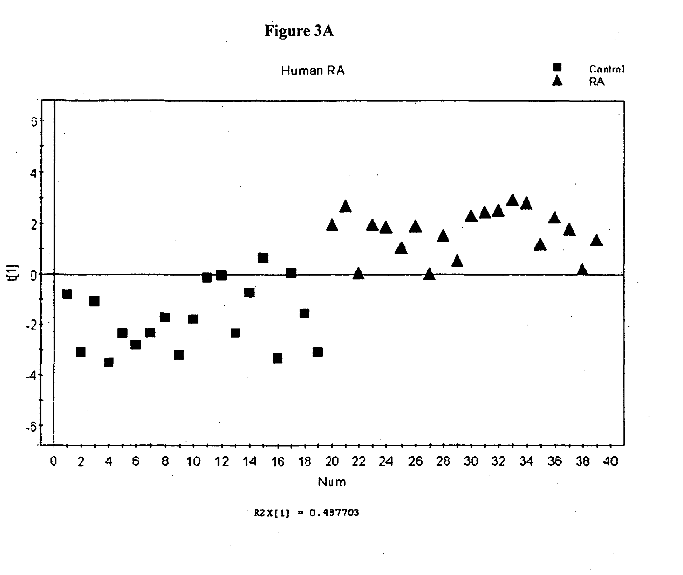

**Figure 3A****.** Scores scatter plot (t[1]) from an OPLS-DA model of human controls and RA patients. The subjects with RA (upper part of the plot, highlighted with triangles) are well separated from the controls (lower part of the plot, highlighted with box symbols). This separation is entirely based on the patterns of endogenous metabolites of the two groups of individuals.

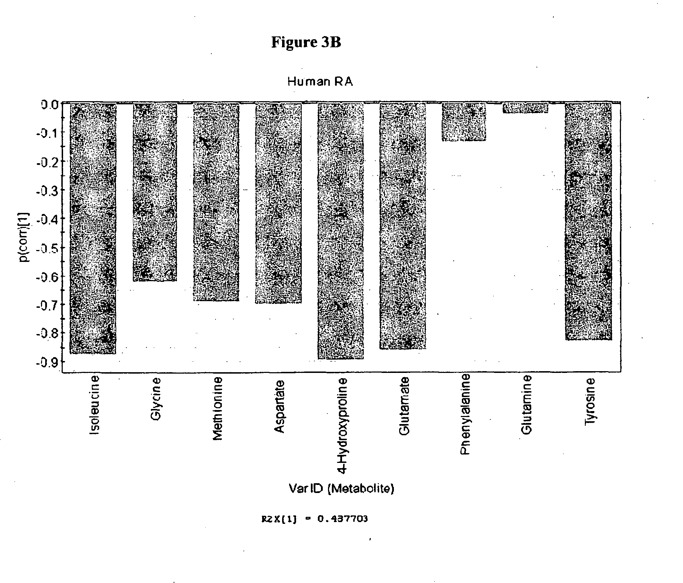

**Figure 3****B.** Loadings column plot (p[1], correlation scaled) from an OPLS-DA model of human controls and RA patients. From this plot the metabolite profile that describes the observed separation between controls and patients diagnosed with RA can be identified. Negative loadings denote endogenous metabolites with decreased levels in subjects with an RA diagnosis. All amino acids are down regulated in the RA group.

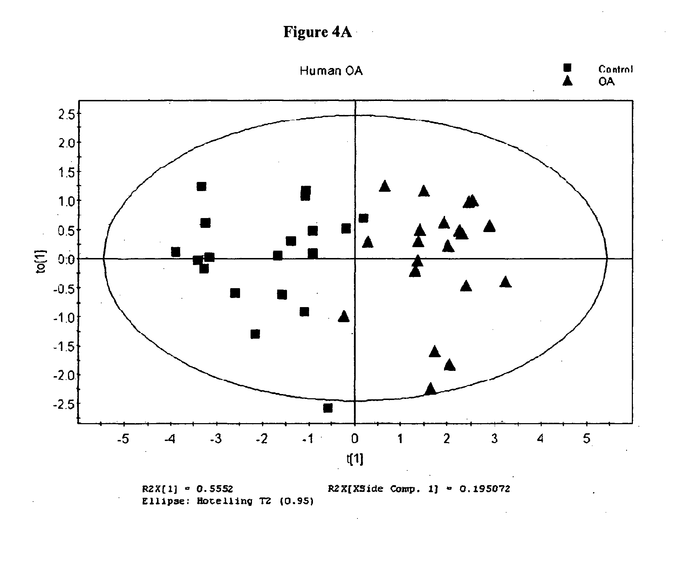

**Figure 4A****.** Scores scatter plot (t[1]/to[1]) from an OPLS-DA model of human controls and OA patients. The subjects with OA (right side in the plot, highlighted with triangles) are well separated from the controls (left side in the plot, highlighted with box symbols). This separation is entirely based on the patterns of endogenous metabolites of the two groups of individuals.

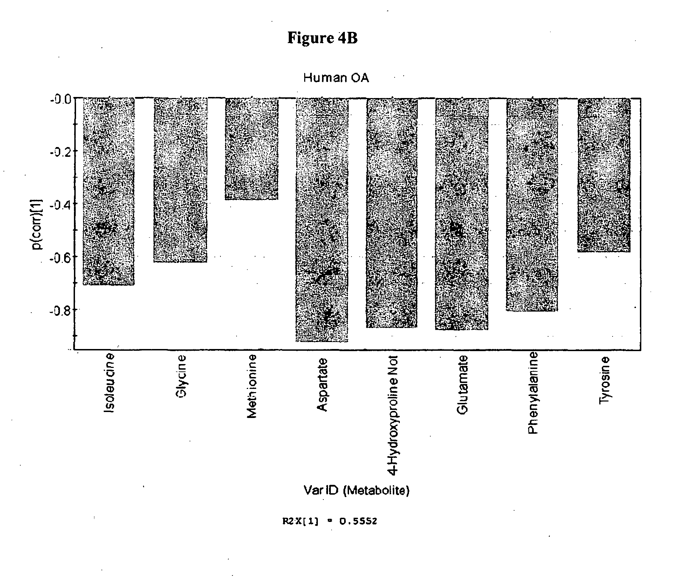

**Figure 4****B.** Loadings column plot (p[1], correlation scaled) from an OPLS-DA model of human controls and OA patients. From this plot the metabolite profile that describes the observed separation between controls and patients diagnosed with OA can be identified. Negative loadings denote endogenous metabolites with decreased levels in subjects with an OA diagnosis. All amino acids are down regulated in the OA group.

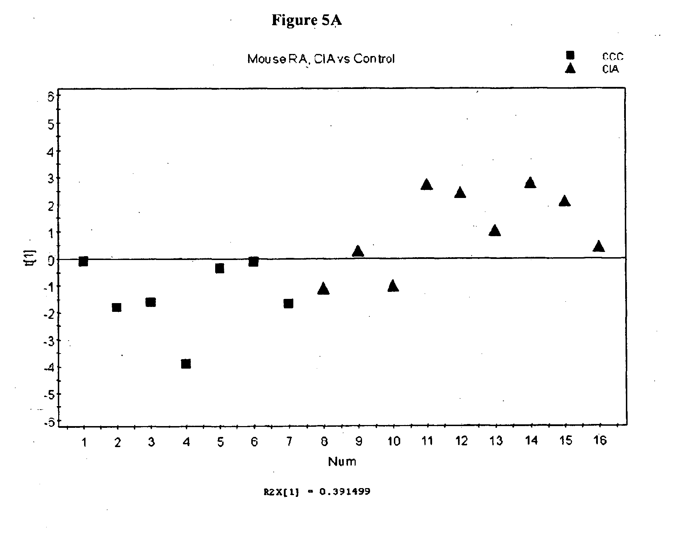

**Figure 5A****.** Scores scatter plot (t[1]/to[1]) from an OPLS-DA model of CIA mice and healthy controls. The mice with CIA (right side in the plot, highlighted with triangles) are well separated from the controls (left side in the plot, highlighted with box symbols). This separation is entirely based on the patterns of endogenous metabolites of the two groups of individuals.

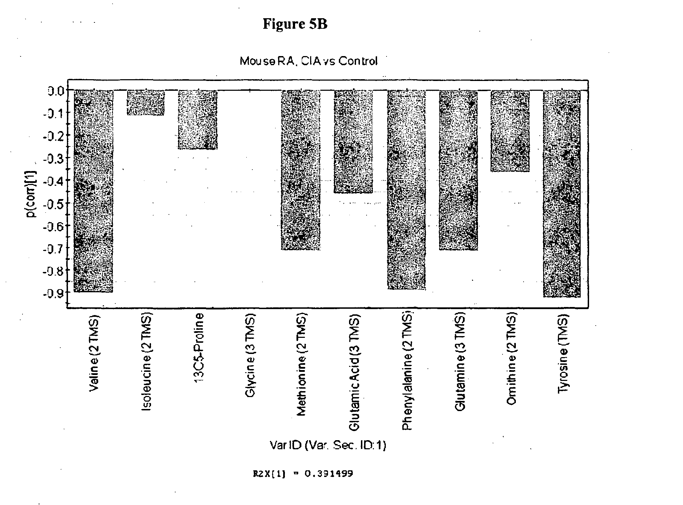

**Figure 5****B.** Loadings column plot (p[1], correlation scaled) from an OPLS-DA model of CIA mice and healthy controls. From this plot the metabolite profile that describes the observed separation between controls and disease group can be identified. Negative loadings denote endogenous metabolites with decreased levels in subjects with CIA. All metabolites are down regulated in the disease group.

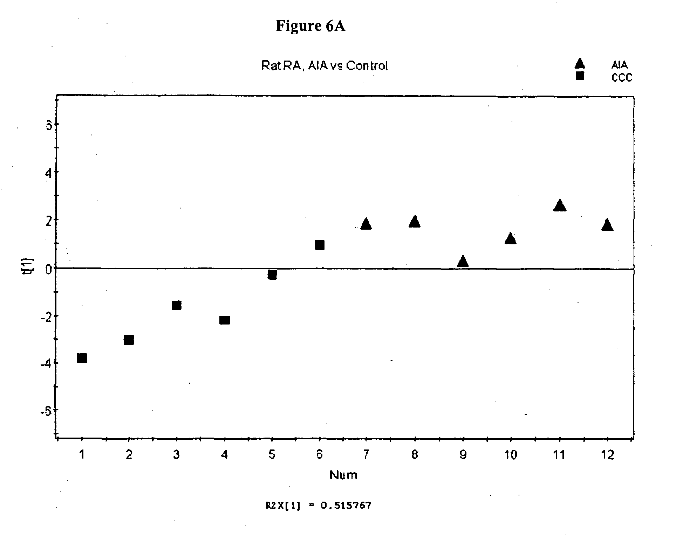

**Figure 6A****.** Scores scatter plot (t[1]/to[1]) from an OPLS-DA model of AIA rats and healthy controls. The AIA rats (right side in the plot, highlighted with triangles) are well separated from the controls (left side in the plot, highlighted with box symbols).

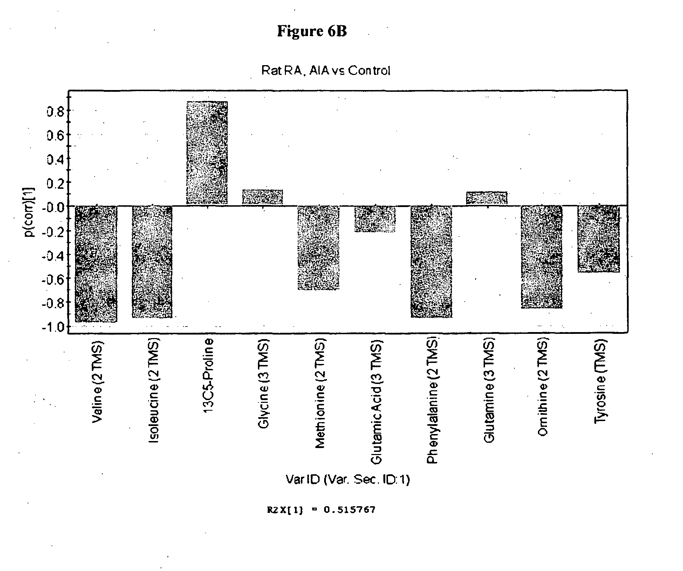

**Figure 6****B.** Loadings column plot (p[1], correlation scaled) from an OPLS-DA model of AIA rats and healthy controls. Many metabolites are down regulated in the disease group.

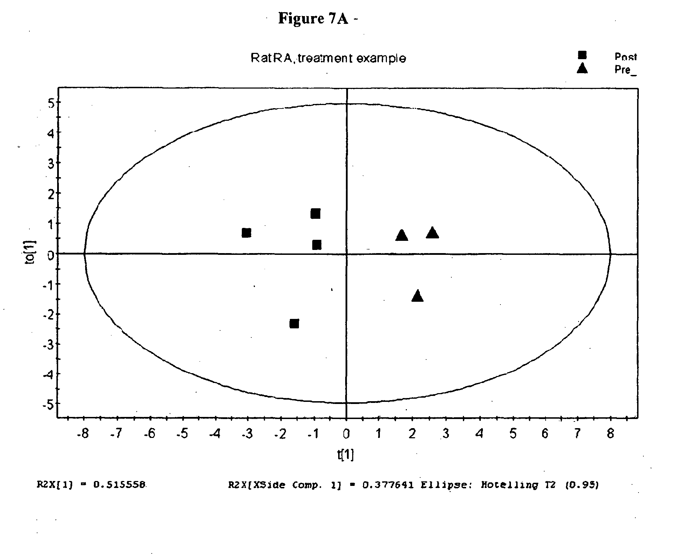

**Figure 7A****.** Scores scatter plot (t[1]/to[1]) from an OPLS-DA model of AIA rats treated with an experimental substance. The untreated AIA animals (right side in the plot, highlighted with triangles) are well separated from the AIA animals who have received treatment with the experimental substance (left side in the plot, highlighted with squares). This separation is entirely based on the patterns of endogenous metabolites of the two groups of individuals.

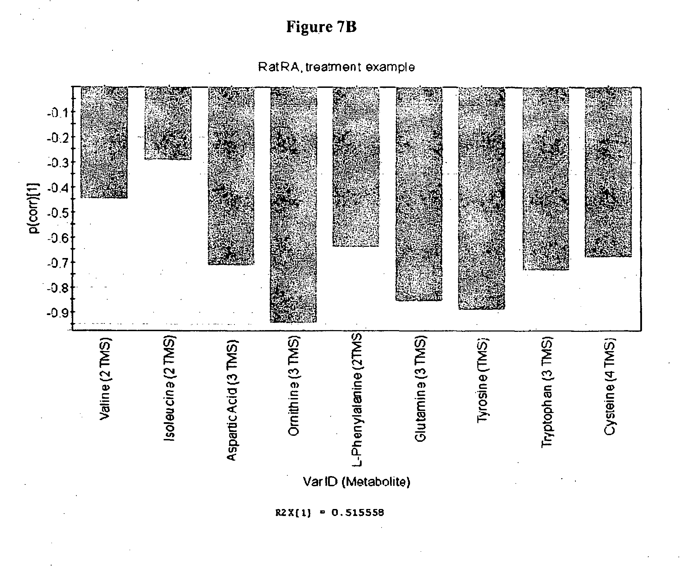

**Figure 7****B.** Loadings column plot (p[1], correlation scaled) from an OPLS-DA model of AIA rats treated with an experimental substance. Almost all metabolites are up regulated in the treated animals, showing efficacy of the administered medicament.

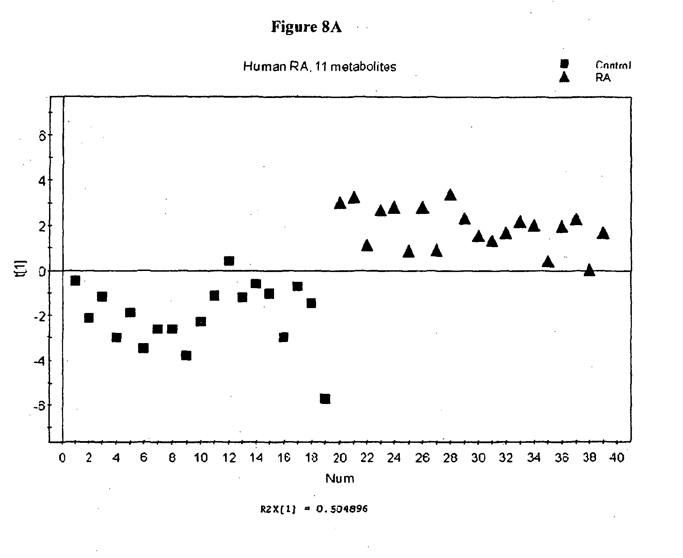

**Figures 8A-17A****.** Scores scatter plot (t[1] vs. to[1]) from OPLS-DA model of various numbers of biomarkers with control subjects vs. subjects with RA. The control samples are highlighted with box symbols and the RA samples are highlighted with triangle symbols. There is excellent separation between healthy controls and diagnosed patients. This separation is entirely based on the patterns of endogenous metabolites of the two groups of individuals.

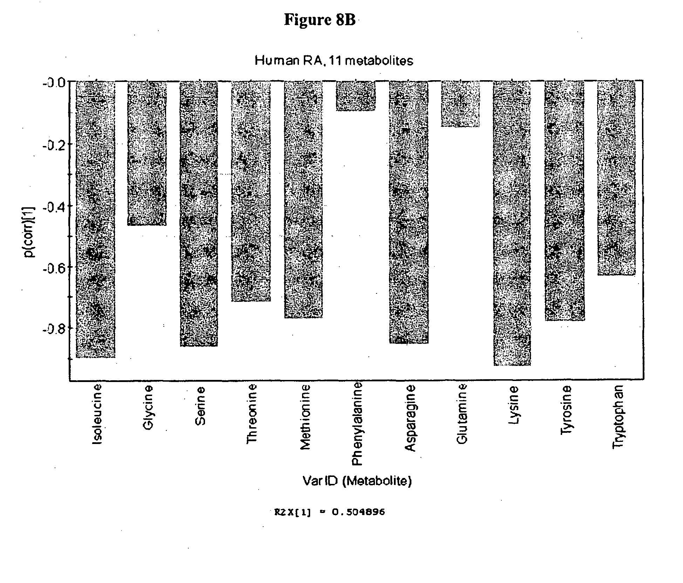

**Figures 8B-17B****.** Plot of correlation scaled loadings from OPLS-DA model of various numbers of biomarkers with control subjects vs. subjects with RA. From this plot the metabolite profile that describes the observed separation between controls and patients diagnosed with RA can be identified. Negative loadings denote biomarkers with decreased levels in subjects with an RA diagnosis.

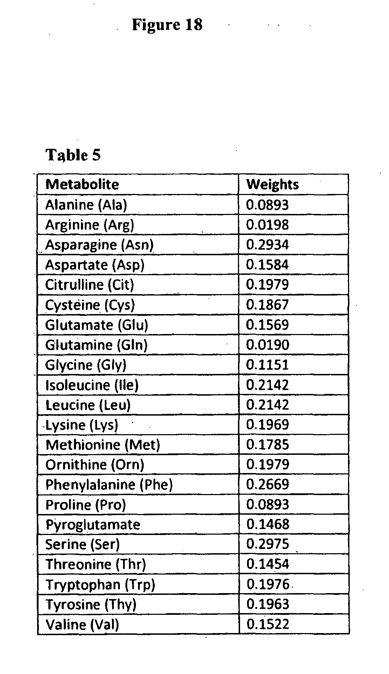

**Figure 18****.** Table 5. Summary of results of comparison between the human RA case and relevant animal models. The weights denote the relative importance of the endogenous metabolites and may be used to provide an interpretation of a sample based on a weighted sum of averages.

The present invention is further defined in the following Examples, in which parts and percentages are by weight and degrees are Celsius, unless otherwise stated. It should be understood that these Examples, while indicating preferred embodiments of the invention, are given by way of illustration only.

### EXAMPLES

### EXAMPLE 1

Validation of animal model as being relevant for human ADs.

### Study design

The human RA and OA samples used consisted of the following blood samples:
- 19 Control patients
- 20 RA patients
- 20 OA patients

Serum samples were collected from 59 persons: 20 of them had the diagnosis RA, 20 of them had the diagnosis OA and 19 blood donors were considered as healthy controls that did not have any diagnosis yet.

The mouse model samples used consist of the following blood samples:
- 7 samples from controls
- 9 samples from subjects with CIA

The rat model samples used consist of the following blood samples:
- 6 samples from controls
- 6 samples from subjects with AIA

### Extraction of metabolites from scrum

Extraction of metabolites from serum samples was essentially performed as the method described by A et al. (2005). 630 µl of MeOH:H₂O (9:1 V:V) including internal standards was added to 70 µl of serum. The solution was vortex mixed for 10 s, kept on ice for 10 min, and then vigorously extracted at a frequency of 30 Hz for 3 min using a MM301 vibration Mill (Retsch GmbH & Co. KG, Haan, Germany. After 120 min on ice, the samples were centrifuged at 19 600 g for 10 min at 4°C. 200 µl of the supernatant was transferred to a GC vial, and 50 µl was transferred to a LC/MS vial and evaporated to dryness.

### GC/TOFMS analysis

Prior GC/MS analysis the samples were derivatised with 30 µL of methoxyamine hydrochloride (15 mg mL-1) in pyridine by shaking for 10 min at 5°C, then incubating them for 16 h at room temperature. The samples were then trimethylsilylated by adding 30 µL of N-Methyl-N-trifluoroacetamide (MSTFA) with 1% TMCS and incubating them for 1 h at room temperature. After silylation, 30 µL of heptane (containing 0.5 µg methyl stearate as internal standard) was added.

0.1 µl of derivatized sample was injected split less by an Agilent 7683 Series Autosampler (Agilent, Atlanta; GA, USA) into an Agilent 6980 GC equipped with a 10 m×0.18 mm ID, fused silica capillary column chemically bonded with 0.18 µm DB5-MS stationary phase (J&W Scientific, Folsom, CA, USA). The injector temperature was set at 270°C. Helium was used as carrier gas at a constant flow rate of 1 ml/min through the column. For every analysis, the purge time was set to 60s at a purge flow rate of 20 ml min-1 and an equilibration time of 1 min. The column temperature was initially kept at 70°C for 2 min, then increased from 70 to 320 °C at 40 °C/min, where it was held for 2 min. The column effluent was introduced into the ion source of a Pegasus III TOFMS (Leco Corp., St Joseph, MI, USA). The transfer line temperature was set at 250°C and ion source temperature at 200°C. Ions were generated by a 70 eV electron beam at a current of 2.0 mA. Masses were acquired from m/z 50 to 800 at a rate of 30 spectra/s, and the acceleration voltage was turned on after a solvent delay of 170 s.

**Data processing of MS-data**To evaluate the extraction protocols, non-processed MS-files from GC/TOFMS and UPLC/MS analysis were exported in NetCDF format to MATLAB software 7.0 (Mathworks, Natick, MA, USA), where all data-pretreatment procedures, such as base-line correction, chromatogram alignment, time-window setting and multivariate curve resolution (MCR) were performed using custom scripts (Jonsson et al. 2005, 2006). Thus the between sample relative metabolite concentrations were obtained. These data were analysed with partial least squares (PLS) (Wold, S. et al., SIAM J. Sci. Statist. Comput. 5: 735-743 (1984); Hoskuldsson, A., J. Chemometr. 9: 91-123 (1995)) and orthogonal PLS (OPLS) (Trygg, J. et al., J. Chemometrics. 16: 119-128 (2002); Trygg, J. et al., J. Chemometrics. 17: 53-64 (2003)) as implemented in SIMCA-P+ software (Umetrics, AB: (2005)) to identify differences between levels of endogenous metabolites in the samples obtained from the groups of individuals, i.e. human RA and control, CIA mouse and controls, and AIA rat and controls.

### Results

The identified metabolites (Table 5, Figures 3A and 3B, 4A and 4B, 5A and 5B, 6A and 6B) consist of amino acids and other metabolites associated with RA and OA, and thus AD in humans. Our OPLS analysis shows that there is a great overlap between the human case and the two animal models and that the metabolites show similar patterns of down regulation in the diseased subjects. The major finding was that it is possible to compare animal models to the human case using this technology and also that it is possible to identify the most relevant model for an indication, in this case AD. This enables validation of the relevance of the animal models for the human case.

### EXAMPLE 2

### Materials and methods

The metabolites were extracted from serum according to standard protocols.

### Clinical samples

The samples used consisted of the following blood samples:
- 19 Control patients
- 20 RA patients

### Procedures

Serum samples were collected from 39 persons: 20 of them had the diagnosis RA and 19 blood donors were considered as healthy controls that did not have any diagnosis yet.

The endogenous metabolites were identified by methods well known in the art and between sample relative metabolite concentrations were obtained. These data were analysed with partial least squares (PLS) (Wold, S. et al., SIAM J. Sci. Statist. Comput. 5: 735-743 (1984); Hoskuldsson, A., J. Chemometr. 9: 91-123 (1995)) and orthogonal PLS (OPLS) (Trygg, J. et al., J. Chemometrics. 16: 119-128 (2002); Trygg, J. et al., J. Chemometrics. 17: 53-64 (2003)) as implemented in SIMCA-P+ software (Umetrics, AB: (2005)) to identify differences between levels of endogenous metabolites in the samples obtained from the two groups of individuals.

### Results

The multivariate data analysis revealed a profile of the endogenous metabolites that separate the subjects that have the diagnosis of RA from the healthy group (Figure 3A). The result was a very clear separation between the two groups.

Using the model parameters, e.g. loadings or weights, the metabolites that are most potent for diagnosis of RA can be identified (Figure 3B).

The results of the study show that the diagnostic properties of the endogenous metabolite profile are highly sensitive and specific for the diagnosis of RA in humans.

The results also show that diagnosis and monitoring of the disease state are intimately related for a person skilled in the art.

### EXAMPLE 3

### Materials and methods

The metabolites were extracted from serum according to standard protocols.

### Clinical samples

The samples used consisted of the following blood samples:
- 19 Control patients
- 20 OA patients

### Procedures

Serum samples were collected from 39 persons: 20 of them had the diagnosis OA and 19 blood donors that were considered as healthy controls that did not have any diagnosis yet.

The endogenous metabolites were identified by methods well known in the art and between sample relative metabolite concentrations were obtained. These data were analysed with partial least squares (PLS) (Wold, S. et al., SIAM J. Sci. Statist. Comput. 5: 735-743 (1984); Hoskuldsson, A., J. Chemometr. 9: 91-123 (1995)) and orthogonal PLS (OPLS) (Trygg, J. et al., J. Chemometrics. 16: 119-128 (2002); Trygg, J. et al., J. Chemometrics. 17: 53-64 (2003)) as implemented in SIMCA-P+ software (Umetrics, AB: (2005)) to identify differences between levels of endogenous metabolites in the samples obtained from the two groups of individuals.

### Results

The multivariate data analysis revealed a profile of the endogenous metabolites that separate the subjects that have the diagnosis of OA from the healthy group (Figure 1). The result was a very clear separation between the two groups.

Using the model parameters, e.g. loadings or weights, the metabolites that were most potent for diagnosis of OA were identified (Figure 2).

The result of the study shows that the diagnostic properties of the endogenous metabolite profile are highly sensitive and specific for the diagnosis of OA in humans. The results also show that diagnosis and monitoring of the disease state are intimately related for a person skilled in the art.

### EXAMPLE 4

Validation of animal model as being relevant for human RA.

### Study design

The mouse model samples used consist of the following blood samples:
- 7 samples from controls
- 9 samples from subjects with CIA

The rat model samples used consist of the following blood samples:
- 6 samples from controls
- 6 samples from subjects with AIA

### Extraction of metabolites from serum

Extraction of metabolites from serum samples was essentially performed as the method described by A et al. (2005). 630 µl of MeOH:H₂O (9:1 V:V) including internal standards was added to 70 µl of serum. The solution was vortex mixed for 10 s, kept on ice for 10 min, and then vigorously extracted at a frequency of 30 Hz for 3 min using a MM301 vibration Mill (Retsch GmbH & Co. KG, Haan, Germany. After 120 min on ice, the samples were centrifuged at 19 600 g for 10 min at 4°C. 200 µl of the supernatant was transferred to a GC vial, and 50 µl was transferred to a LC/MS vial and evaporated to dryness.

### GC/TOFMS analysis

Prior GC/MS analysis the samples were derivatised with 30 µL of methoxyamine hydrochloride (15 mg mL-1) in pyridine by shaking for 10 min at 5°C, then incubating them for 16 h at room temperature. The samples were then trimethylsilylated by adding 30 µL of N-Methyl-N-trifluoroacetamide (MSTFA) with 1% TMCS and incubating them for 1 h at room temperature. After silylation, 30 µL of heptane (containing 0.5 µg methyl stearate as internal standard) was added. 0.1 µl of derivatized sample was injected split less by an Agilent 7683 Series Autosampler (Agilent, Atlanta; GA, USA) into an Agilent 6980 GC equipped with a 10 m×0.18 mm ID, fused silica capillary column chemically bonded with 0.18 µm DB5-MS stationary phase (J&W Scientific, Folsom, CA, USA). The injector temperature was set at 270°C. Helium was used as carrier gas at a constant flow rate of 1 ml/min through the column. For every analysis, the purge time was set to 60s at a purge flow rate of 20 ml min-1 and an equilibration time of 1 min. The column temperature was initially kept at 70°C for 2 min, then increased from 70 to 320 °C at 40 °C/min, where it was held for 2 min. The column effluent was introduced into the ion source of a Pegasus III TOFMS (Leco Corp., St Joseph, MI, USA). The transfer line temperature was set at 250°C and ion source temperature at 200°C. Ions were generated by a 70 eV electron beam at a current of 2.0 mA. Masses were acquired from m/z 50 to 800 at a rate of 30 spectra/s, and the acceleration voltage was turned on after a solvent delay of 170 s.

**Data processing of MS-data**To evaluate the extraction protocols, non-processed MS-files from GC/TOFMS and UPLC/MS analysis were exported in NetCDF format to MATLAB software 7.0 (Mathworks, Natick, MA, USA), where all data-pretreatment procedures, such as base-line correction, chromatogram alignment, time-window setting and multivariate curve resolution (MCR) were performed using custom scripts (Jonsson et al. 2005, 2006). Thus the between sample relative metabolite concentrations were obtained. These data were analysed with partial least squares (PLS) (Wold, S. et al., SIAM J. Sci. Statist. Comput. 5: 735-743 (1984); Hoskuldsson, A., J. Chemometr. 9: 91-123 (1995)) and orthogonal PLS (OPLS) (Trygg, J. et al., J. Chemometrics. 16: 119-128 (2002); Trygg, J. et al., J. Chemometrics. 17: 53-64 (2003)) as implemented in SIMCA-P+ software (Umetrics, AB: (2005)) to identify differences between levels of endogenous metabolites in the samples obtained from the groups of individuals, i.e. human RA and control, CIA mouse and controls, and AIA rat and controls.

### Results

The identified metabolites (Table 5, Figures 3A and 3B, 4A and 4B, 5A and 5B, 6A and 6B) consist of amino acids and other metabolites associated with RA and OA, and thus AD in humans. Our OPLS analysis shows that there is a great overlap between the human case and the two animal models and that the metabolites show similar patterns of down regulation in the diseased subjects. The major finding was that it is possible to compare animal models to the human case using this technology and also that it is possible to identify the most relevant model for an indication, in this case AD. This enables validation of the relevance of the animal models for the human case.

### EXAMPLE 5

### Treatment of RA in an AIA rat model with an experimental substance (Arginine)

### Study design

AIA was induced in three Dark Agouti (DA) rats and the rats were then given treatment with an experimental substance.

The rat model samples used consist of the following blood samples:
- 3 samples from AIA DA rats
- 4 samples (one of the rats was sampled twice) from AIA DA rats treated with the experimental substance.

### Extraction of metabolites from serum

Extraction of metabolites from serum samples was essentially performed as the method described by A et al. (2005). 630 µl of MeOH:H₂O (9:1 V:V) including internal standards was added to 70 µl of serum. The solution was vortex mixed for 10 s, kept on ice for 10 min, and then vigorously extracted at a frequency of 30 Hz for 3 min using a MM301 vibration Mill (Retsch GmbH & Co. KG, Haan, Germany. After 120 min on ice, the samples were centrifuged at 19 600 g for 10 min at 4°C. 200 µl of the supernatant was transferred to a GC vial, and 50 µl was transferred to a LC/MS vial and evaporated to dryness.

### GC/TOFMS analysis

Prior GC/MS analysis the samples were derivatised with 30 µL of methoxyamine hydrochloride (15 mg mL-1) in pyridine by shaking for 10 min at 5°C, then incubating them for 16 h at room temperature. The samples were then trimethylsilylated by adding 30 µL of N-Methyl-N-trifluoroacetamide (MSTFA) with 1% TMCS and incubating them for 1 h at room temperature. After silylation, 30 µL of heptane (containing 0.5 µg methyl stearate as internal standard) was added. 0.1 µl of derivatized sample was injected split less by an Agilent 7683 Series Autosampler (Agilent, Atlanta; GA, USA) into an Agilent 6980 GC equipped with a 10 m×0.18 mm ID, fused silica capillary column chemically bonded with 0.18 µm DB5-MS stationary phase (J&W Scientific, Folsom, CA, USA). The injector temperature was set at 270°C. Helium was used as carrier gas at a constant flow rate of 1 ml/min through the column. For every analysis, the purge time was set to 60s at a purge flow rate of 20 ml min-1 and an equilibration time of 1 min. The column temperature was initially kept at 70°C for 2 min, then increased from 70 to 320 °C at 40 °C/min, where it was held for 2 min. The column effluent was introduced into the ion source of a Pegasus III TOFMS (Leco Corp., St Joseph, MI, USA). The transfer line temperature was set at 250°C and ion source temperature at 200°C. Ions were generated by a 70 eV electron beam at a current of 2.0 mA. Masses were acquired from m/z 50 to 800 at a rate of 30 spectra/s, and the acceleration voltage was turned on after a solvent delay of 170 s.

**Data processing of MS-data**To evaluate the extraction protocols, non-processed MS-files from GC/TOFMS and UPLC/MS analysis were exported in NetCDF format to MATLAB software 7.0 (Mathworks, Natick, MA, USA), where all data-pretreatment procedures, such as base-line correction, chromatogram alignment, time-window setting and multivariate curve resolution (MCR) were performed using custom scripts (Jonsson et al. 2005, 2006). Thus the between sample relative metabolite concentrations were obtained. These data were analysed with partial least squares (PLS) (Wold, S. et al., SIAM J. Sci. Statist. Comput. 5: 735-743 (1984); Hoskuldsson, A., J. Chemometr. 9: 91-123 (1995)) and orthogonal PLS (OPLS) (Trygg, J. et al., J. Chemometrics. 16: 119-128 (2002); Trygg, J. et al., J. Chemometrics. 17: 53-64 (2003)) as implemented in SIMCA-P+ software (Umetrics, AB: (2005)) to identify differences between levels of endogenous metabolites in the samples obtained from the AIA DA rats and the treated individuals.

### Data analysis

Data describing the between sample relative metabolite concentrations were obtained. These data were analysed with partial least squares (PLS) (Wold, S. et al., SIAM J. Sci. Statist. Comput. 5: 735-743 (1984); Hoskuldsson, A., J. Chemometr. 9: 91-123 (1995)) and orthogonal PLS (OPLS) (Trygg, J. et al., J. Chemometrics. 16: 119-128 (2002); Trygg, J. et al., J. Chemometrics. 17: 53-64 (2003)) as implemented in SIMCA-P+ software (Umetrics, AB: (2005)) to identify differences between levels of endogenous metabolites in the samples obtained from the groups of individuals, i.e. human RA and control, CIA mouse and controls, and AIA rat and controls.

### Results

The identified metabolites (Figures 7A and 7B) consist of amino acids associated with AD in humans and relevant animal models. Our OPLS analysis shows that the described technology enabled identification of important metabolic changes in the AIA rat after the substance had been administered. The administered substance resulted in up regulation of many of the metabolites associated with AD in humans and relevant animal models. The applied technology for serum analysis of metabolites is sensitive and specific for measuring the efficacy of the administered medicament for the indication AD using this approach.

### EXAMPLE 6

This example illustrates the potency of the active compound and its therapeutically active acid addition salts for treatment of mental disorders.

### Study design

AIA was induced in a total of 21 Lewis rats were included in the treatment study. Samples used consist of the following blood samples:
- 22 samples from AIA Lewis rats (one of the rats was sampled twice)
- 26 samples from treated AIA Lewis rats (five of the rats was sampled twice)

Different treatments were distributed in the AIA subjects as follows:
- 5 subjects received 1mg active compound
- 5 subjects received 3mg active compound
- 6 subjects received 10mg active compound
- 5 subjects received 1mg methotrexate

### Extraction of metabolites from serum

Extraction of metabolites from serum samples was essentially performed as the method described by A et al. (2005). 630 µl of MeOH:H₂O (9:1 V:V) including internal standards was added to 70 µl of serum. The solution was vortex mixed for 10 s, kept on ice for 10 min, and then vigorously extracted at a frequency of 30 Hz for 3 min using a MM301 vibration Mill (Retsch GmbH & Co. KG, Haan, Germany. After 120 min on ice, the samples were centrifuged at 19 600 g for 10 min at 4°C. 200 µl of the supernatant was transferred to a GC vial, and 50 µl was transferred to a LC/MS vial and evaporated to dryness.

GC/TOFMS analysis and the Data processing of MS-data was carried out as in Example 1 above.

### Results

The levels of the metabolites, consisting of amino acids and other metabolites associated with T1D, RA and OA, and thus AD in humans, that were identified as relevant endogenous metabolites for detection of risk of AD onset, detection of AD and monitoring of AD progression (Table 5, Figures 3A and 3B, 4A and 4B, 5A and 5B, 6A and 6B) were significantly affected by the active compound treatment. Our OPLS analysis shows that the metabolites show similar patterns of up regulation in the subjects treated with the active compound. The major finding was that it is possible to restore levels of metabolites associated with the indication AD in animal models. This has enabled validation of the relevance of the active compound treatment for prophylactic treatment of AD, treatment to delay or prevent onset of AD, and treatment of AD in animal models , and also for the human case.

### EXAMPLE 7

### Materials and methods

The metabolites were extracted from serum according to standard protocols.

### Clinical samples

The samples used consisted of the following blood samples:
- 19 Control patients
- 20 RA patients

### Procedures

Serum samples were collected from 39 persons: 20 of them had the diagnosis RA and 19 blood donors were considered as healthy controls that did not have any diagnosis yet.

The endogenous metabolites were identified by methods well known in the art and between sample relative metabolite concentrations were obtained. These data were analysed with partial least squares (PLS) (Wold, S. et al., SIAM J. Sci. Statist. Comput. 5: 735-743 (1984); Hoskuldsson, A., J. Chemometr. 9: 91-123 (1995)) and orthogonal PLS (OPLS) (Trygg, J. et al., J. Chemometrics. 16: 119-128 (2002); Trygg, J. et al., J. Chemometrics. 17: 53-64 (2003)) as implemented in SIMCA-P+ software (Umetrics, AB: (2005)) to identify differences between levels of endogenous metabolites in the samples obtained from the two groups of individuals.

### Results

The multivariate data analysis revealed a profile of the endogenous metabolites that separate the subjects that have the diagnosis of RA from the healthy group (Figure 8A). The result was a very clear separation between the two groups.

Using the model parameters, e.g. loadings or weights, the metabolites that are most potent for diagnosis of RA can be identified (Figure 8B).

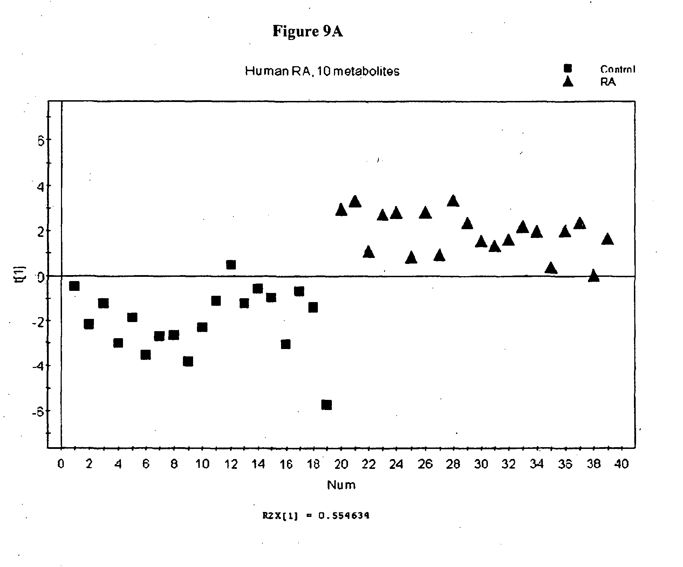

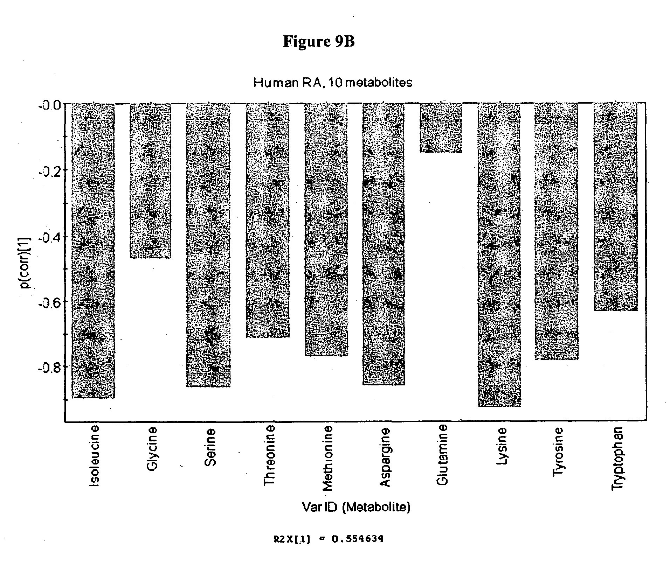

Furthermore, all of the endogenous metabolites do not have to be used to enable separation of subjects that have the diagnosis RA from the healthy group (Figure 9A). The result was a very clear separation between the two groups. Using the model parameters, e.g. loadings or weights, the metabolites that are most potent for diagnosis of RA can be identified (Figure 9B).

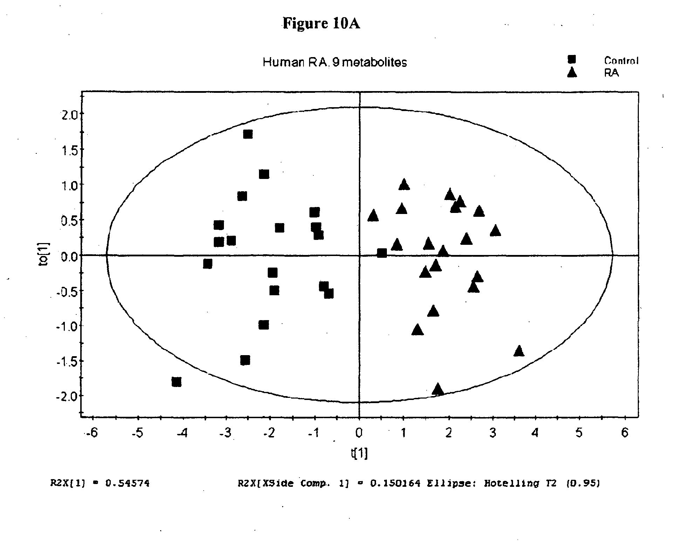

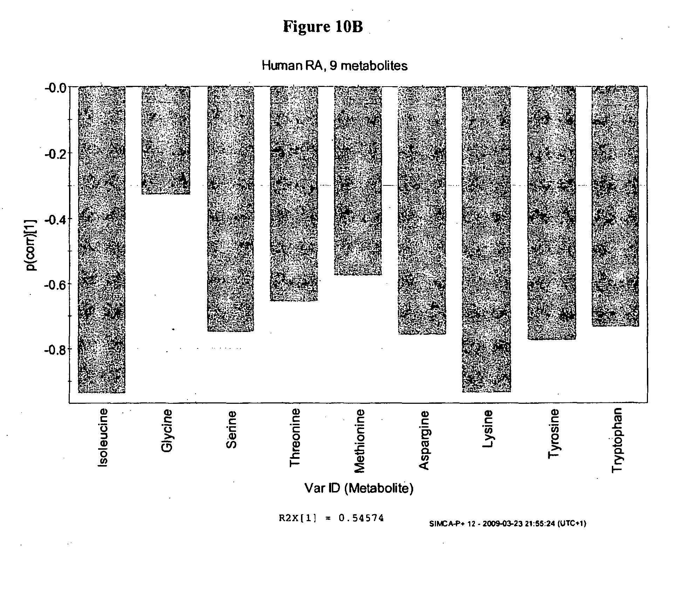

Furthermore, all of the endogenous metabolites do not have to be used to enable separation of subjects that have the diagnosis RA from the healthy group (Figure 10A). The result was a very clear separation between the two groups. Using the model parameters, e.g. loadings or weights, the metabolites that are most potent for diagnosis of RA can be identified (Figure 10B).

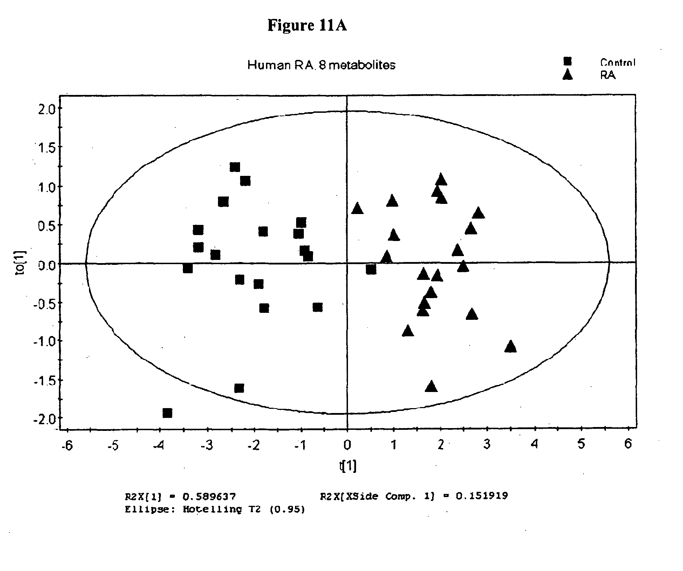

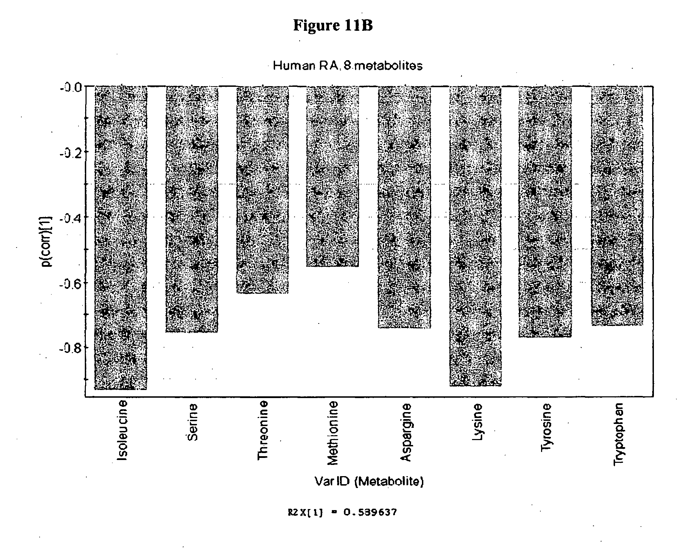

Furthermore, all of the endogenous metabolites do not have to be used to enable separation of subjects that have the diagnosis RA from the healthy group (Figure 11A). The result was a very clear separation between the two groups. Using the model parameters, e.g. loadings or weights, the metabolites that are most potent for diagnosis of RA can be identified (Figure 11B).

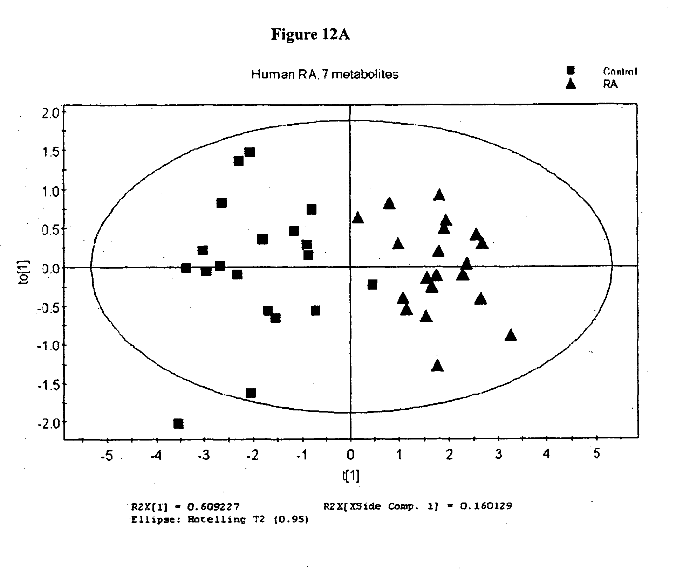

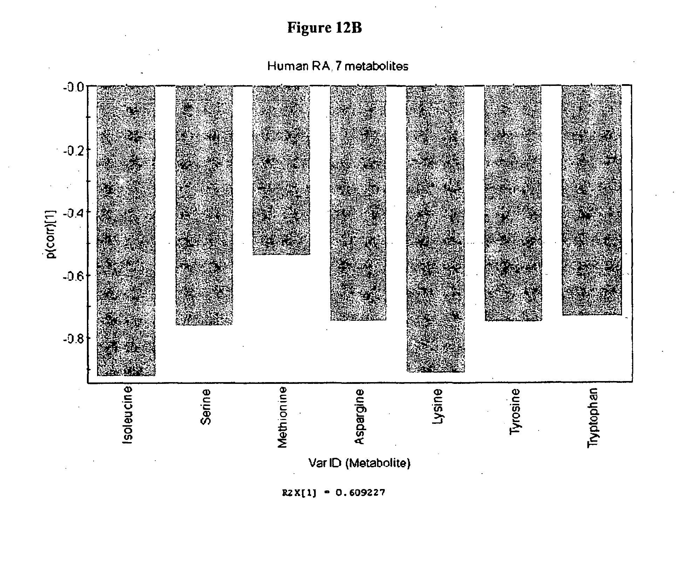

Furthermore, all of the endogenous metabolites do not have to be used to enable separation of subjects that have the diagnosis RA from the healthy group (Figure 12A). The result was a very clear separation between the two groups. Using the model parameters, e.g. loadings or weights, the metabolites that are most potent for diagnosis of RA can be identified (Figure 12B).

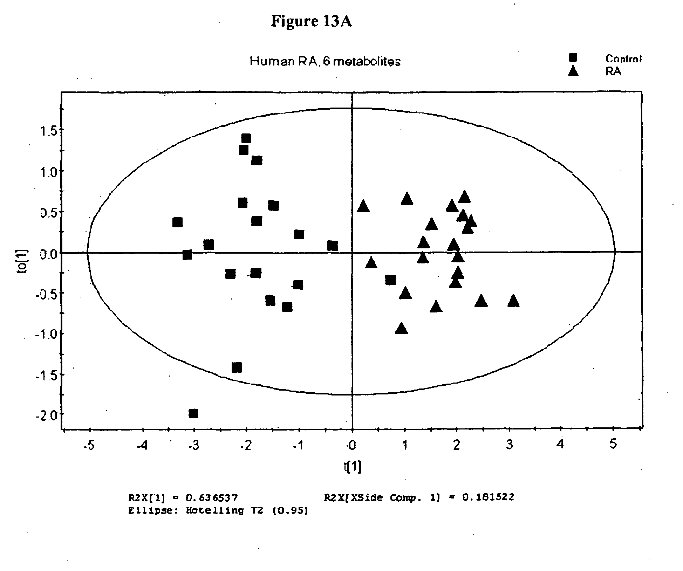

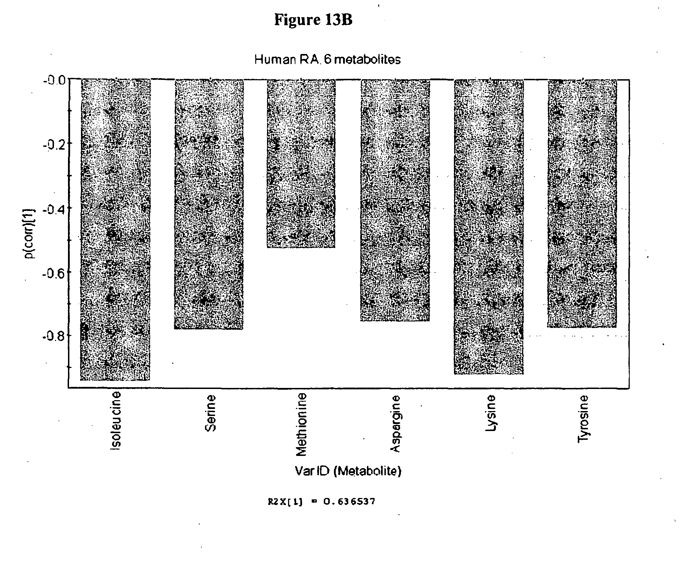

Furthermore, all of the endogenous metabolites do not have to be used to enable separation of subjects that have the diagnosis RA from the healthy group (Figure 13A). The result was a very clear separation between the two groups. Using the model parameters, e.g. loadings or weights, the metabolites that are most potent for diagnosis of RA can be identified (Figure 13B).

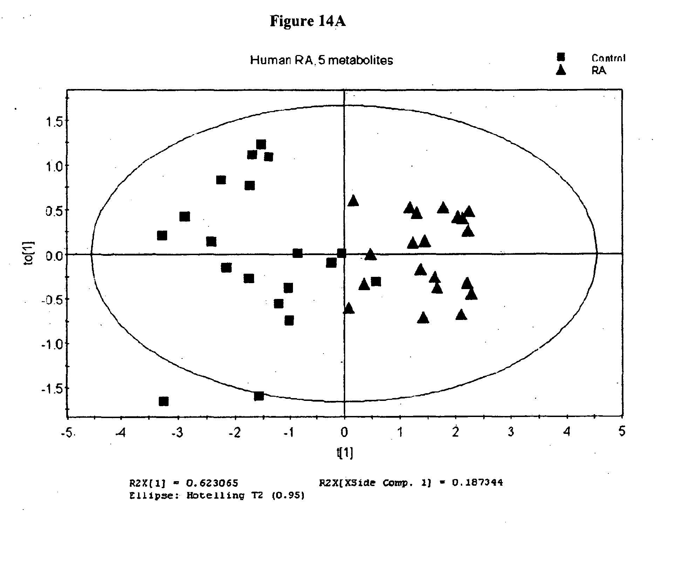

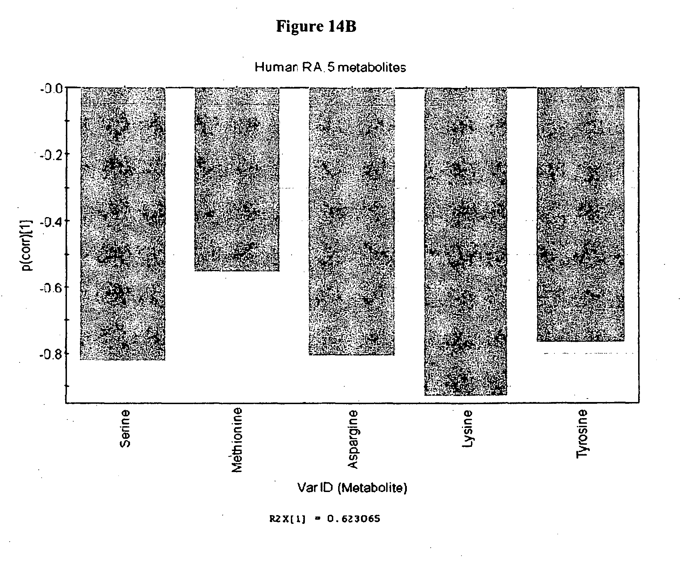

Furthermore, all of the endogenous metabolites do not have to be used to enable separation of subjects that have the diagnosis RA from the healthy group (Figure 14A). The result was a very clear separation between the two groups. Using the model parameters, e.g. loadings or weights, the metabolites that are most potent for diagnosis of RA can be identified (Figure 14B).

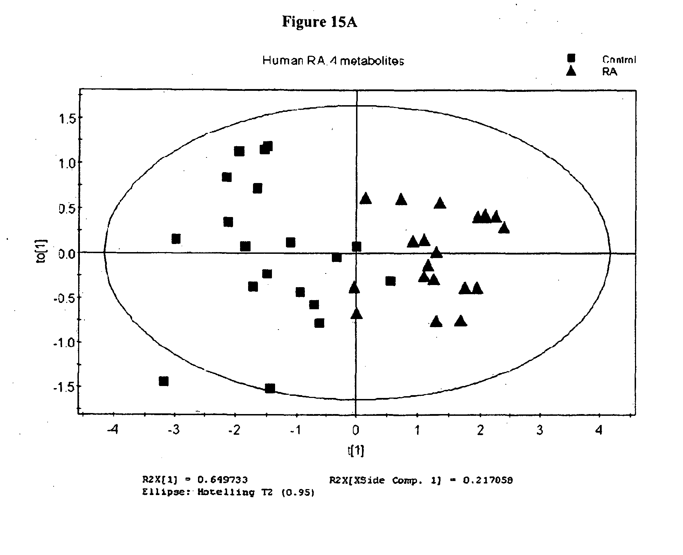

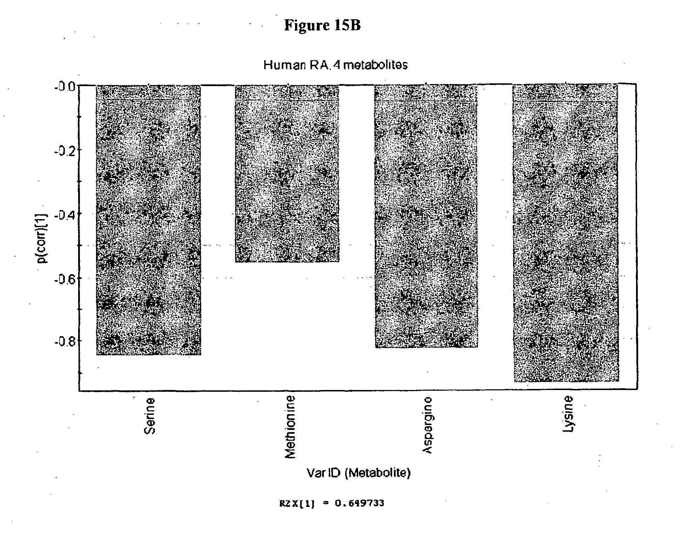

Furthermore, all of the endogenous metabolites do not have to be used to enable separation of subjects that have the diagnosis RA from the healthy group (Figure 15A). The result was a very clear separation between the two groups. Using the model parameters, e.g. loadings or weights, the metabolites that are most potent for diagnosis of RA can be identified (Figure 15B).

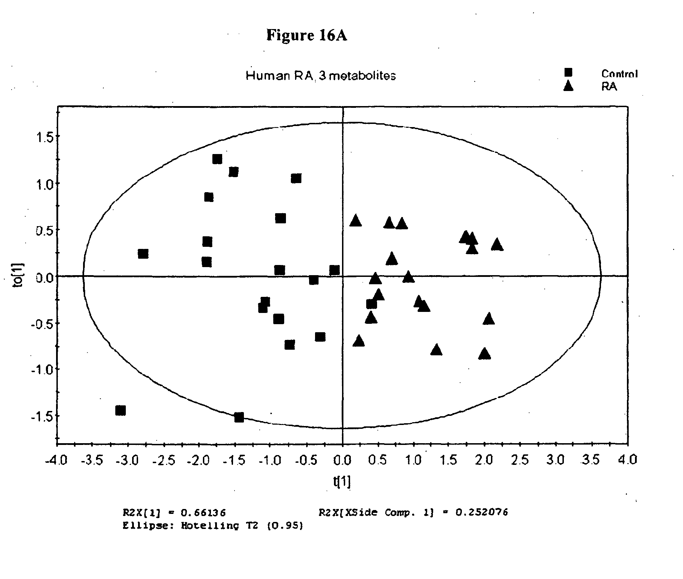

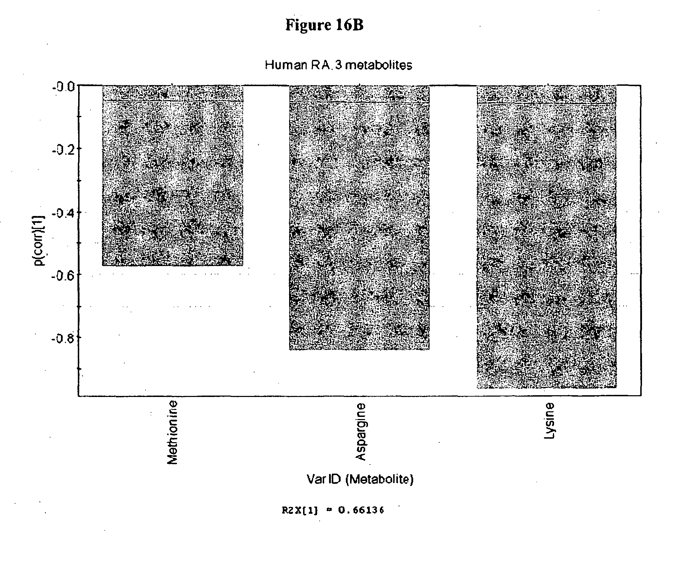

Furthermore, all of the endogenous metabolites do not have to be used to enable separation of subjects that have the diagnosis RA from the healthy group (Figure 16A). The result was a very clear separation between the two groups. Using the model parameters, e.g. loadings or weights, the metabolites that are most potent for diagnosis of RA can be identified (Figure 16B).

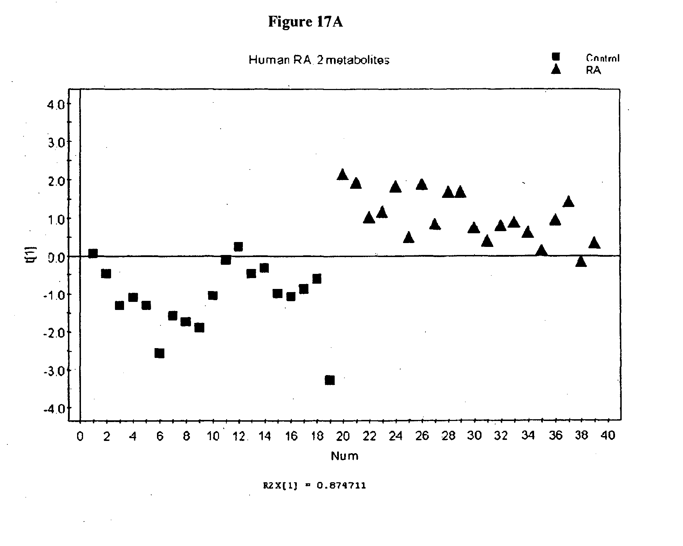

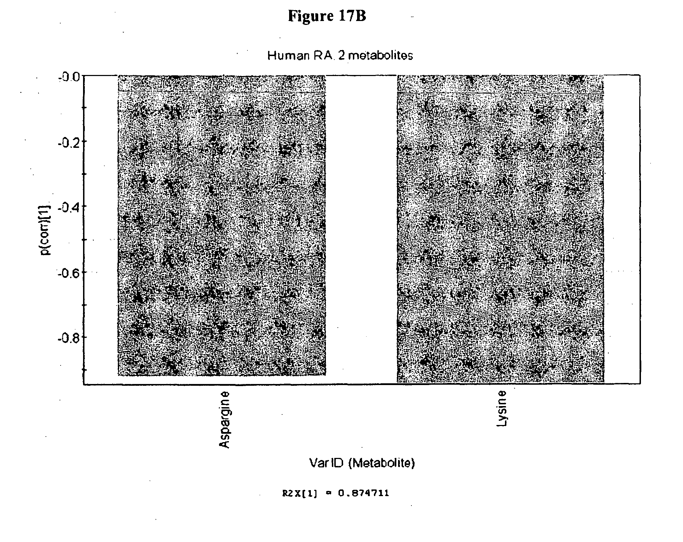

Furthermore, all of the endogenous metabolites do not have to be used to enable separation of subjects that have the diagnosis RA from the healthy group (Figure 17A). The result was a very clear separation between the two groups. Using the model parameters, e.g. loadings or weights, the metabolites that are most potent for diagnosis of RA can be identified (Figure 17B).

The results of the study show that the diagnostic properties of the endogenous metabolite profile are highly sensitive and specific for RA in humans. Furthermore, it is the combination of specific endogenous metabolites that offer the enhanced diagnosis properties.

The results also show that diagnosis and monitoring of the disease state are intimately related for a person skilled in the art.

## Claims

1. An *in vitro* method for the detection of a disturbance in the metabolic profile of endogenous metabolites in a subject caused by rheumatoid arthritis (RA), the method comprising:
(i) measuring the levels of N endogenous metabolites in a biological sample obtained from the subject, wherein N is 11, in order to produce a metabolic profile of the N endogenous metabolites in that subject;
(ii) comparing the measured levels of the N endogenous metabolites with the corresponding levels of the endogenous metabolites in a biological sample obtained from a control;
wherein the N endogenous metabolites are:
EM1 Phenylalanine
EM2 Tyrosine
EM3 Isoleucine
EM8 Glycine
EM9 Glutamine
EM10 Methionine
EM14 Lysine
EM15 Asparagine
EM16 Serine
EM17 Tryptophan
EM18 Threonine
wherein the disturbance in the metabolic profile is one wherein there is a decrease in the level of each of the N measured endogenous metabolites in the biological sample obtained from the subject compared to the corresponding levels of endogenous metabolites in the biological sample obtained from the control,
and wherein the disturbance in the metabolic profile is due to RA in the subject.

2. An *in vitro* method for the detection of a disturbance in the metabolic profile of endogenous metabolites in a subject caused by rheumatoid arthritis (RA), the method comprising:
(i) measuring the levels of N endogenous metabolites in a biological sample obtained from the subject, wherein N is 11, in order to produce a metabolic profile of the N endogenous metabolites in that subject;
(ii) comparing the measured levels of the N endogenous metabolites with the corresponding levels of the endogenous metabolites in a biological sample previously obtained from the subject;
wherein the N endogenous metabolites are:
EM1 Phenylalanine
EM2 Tyrosine
EM3 Isoleucine
EM8 Glycine
EM9 Glutamine
EM10 Methionine
EM14 Lysine
EM15 Asparagine
EM16 Serine
EM17 Tryptophan
EM18 Threonine
wherein the disturbance in the metabolic profile is one wherein there is a decrease in the level of each of the N measured endogenous metabolites in the biological sample obtained from the subject compared to the corresponding levels of endogenous metabolites in the biological sample previously obtained from the subject;
and wherein the disturbance in the metabolic profile is due to RA in the subject.

3. A method for monitoring the rate of decline or rate of improvement or rate of restoring of a healthy metabolic profile in a subject, comprising a method as defined in claim 2,
and additionally comprising the step of dividing the decrease of endogenous metabolites by the time interval between the taking of the first (i.e. previous) and second samples from the subject.

4. A method as claimed in any one of the preceding claims, wherein the biological sample is a biological fluid, preferably blood or synovial fluid.

5. A method as claimed in any one of the preceding claims, wherein the subject is a warm-blooded animal.

6. A method as claimed in claim 5, wherein the subject is a mammal, preferably a human.

7. A method as claimed in any one of the preceding claims, wherein one or more of the levels of the N endogenous metabolites are measured by chromatographic or spectroscopic means, preferably wherein the chromatographic or spectroscopic methods is Fourier Transform Infra-Red (FT-IR) spectroscopy, nuclear magnetic resonance (NMR) spectroscopy, high-performance liquid chromatography (HPLC), liquid chromatography-mass spectrometry (LC-MS) or gas chromatography-mass spectrometry (GC-MS).

8. A method as claimed in any one of the preceding claims, wherein one or more of the levels of the N endogenous metabolites are measured by spectroscopic techniques used in conjunction with chemometric methods, preferably wherein the chemometric method is principal component analysis (PCA), partial least squares projections to latent structures (PLS), orthogonal PLS (OPLS), PLS discriminant analysis (PLS-DA) or orthogonal PLS-DA (OPLS-DA).

9. A method as claimed in any one of the preceding claims, wherein:
(a) one or more of the endogenous metabolites are measured by taking a multi-wavelength spectroscopy measurement; or
(b) one or more of the endogenous metabolites are chemically transformed (i.e. derivatized) before they are measured.

10. A method as claimed in any one of claims 1 to 9, wherein one or more of the endogenous metabolites are measured by using reagents which individually bind to the individual endogenous metabolites, and which are then detected.

11. A method as claimed in any one of the preceding claims wherein the decreases in the levels of the endogenous metabolites (between the subject and the control samples or between the samples taken at different time intervals from the subject) are significant decreases.

12. N-(2-chloro-3,4-dimethoxybenzylideneamino) guanidine, either as the free base or in salt form, for use in treating rheumatoid arthritis in a subject, wherein the subject is one in which a disturbance in the metabolic profile of endogenous metabolites caused by rheumatoid arthritis has been detected by a method as claimed in any one of claims 1 to 11.

## Patentansprüche

1. *In vitro* Verfahren zum Nachweis einer Störung im Stoffwechselprofil endogener Stoffwechselprodukte bei einer Zielperson, die durch rheumatoide Arthritis (RA) hervorgerufen wird, wobei das Verfahren umfasst:
(i) Messen der Gehalte an N endogenen Stoffwechselprodukten in einer biologischen Probe, die von der Zielperson erhalten wurde, wobei N 11 ist, um ein Stoffwechselprofil der N endogenen Stoffwechselprodukte in der Zielperson zu erstellen;
(ii) Vergleichen der gemessenen Gehalte der N endogenen Stoffwechselprodukte mit den entsprechenden Gehalten der endogenen Stoffwechselprodukte in einer biologischen Probe, die von einer Kontrolle erhalten wurde;
wobei die N endogenen Stoffwechselprodukte sind:
EM1 Phenylalanin
EM2 Tyrosin
EM3 Isoleucin
EM8 Glycin
EM9 Glutamin
EM10 Methionin
EM14 Lysin
EM15 Asparagin
EM16 Serin
EM17 Tryptophan
EM18 Threonin
wobei die Störung im Stoffwechselprofil dergestalt ist, dass eine Verringerung des Gehalts jedes der N gemessenen Stoffwechselprodukte in der biologischen Probe, die von der Zielperson erhalten wurde, vorliegt verglichen mit den entsprechenden Gehalten der endogenen Metaboliten in der biologischen Probe, die von der Kontrolle erhalten wurde,
und wobei die Störung im Stoffwechselprofil aufgrund von RA bei der Zielperson vorliegt.

2. *In vitro* Verfahren zum Nachweis einer Störung im Stoffwechselprofil endogener Stoffwechselprodukte bei einer Zielperson, die durch rheumatoide Arthritis (RA) hervorgerufen wird, wobei das Verfahren umfasst:
(i) Messen der Gehalte an N endogenen Stoffwechselprodukten in einer biologischen Probe, die von der Zielperson erhalten wurde, wobei N 11 ist, um ein Stoffwechselprofil der N endogenen Stoffwechselprodukte in der Zielperson zu erstellen;
(ii) Vergleichen der gemessenen Gehalte der N endogenen Stoffwechselprodukte mit den entsprechenden Gehalten der endogenen Stoffwechselprodukte in einer biologischen Probe, die früher von der Zielperson erhalten wurde;
wobei die N endogenen Stoffwechselprodukte sind:
EM1 Phenylalanin
EM2 Tyrosin
EM3 Isoleucin
EM8 Glycin
EM9 Glutamin
EM10 Methionin
EM14 Lysin
EM15 Asparagin
EM16 Serin
EM17 Tryptophan
EM18 Threonin
wobei die Störung im Stoffwechselprofil dergestalt ist, dass eine Verringerung des Gehalts jedes der N gemessenen Stoffwechselprodukte in der biologischen Probe, die von der Zielperson erhalten wurde, vorliegt verglichen mit den entsprechenden Gehalten der endogenen Metaboliten in der biologischen Probe, die von der Person früher erhalten wurde,
und wobei die Störung im Stoffwechselprofil aufgrund von RA bei der Zielperson vorliegt.

3. Verfahren zur Überwachung der Verfallsrate oder der Verbesserungsrate oder der Wiederherstellungsrate eines gesunden Stoffwechselprofils in einer Zielperson, das ein Verfahren wie in Anspruch 2 definiert umfasst,
sowie zusätzlich den Schritt des Teilens der Abnahme endogener Stoffwechselprodukte durch das Zeitintervall zwischen der Entnahme der ersten (i.e. früheren) und der zweiten Proben von der Zielperson umfasst.

4. Verfahren wie in einem der vorherigen Ansprüche beansprucht, wobei die biologische Probe eine biologische Flüssigkeit, vorzugsweise Blut oder Synovialflüssigkeit ist.

5. Verfahren wie in einem der vorherigen Ansprüche beansprucht, wobei die Zielperson ein warmblütiges Tier ist.

6. Verfahren wie in Anspruch 5 beansprucht, wobei die Zielperson ein Säugetier, vorzugsweise ein Mensch ist.

7. Verfahren wie in einem der vorherigen Ansprüche beansprucht, wobei einer oder mehrere der Gehalte der N endogenen Stoffwechselprodukte durch chromatographische oder spektroskopische Mittel gemessen werden, vorzugsweise wobei das chromatographische oder spektroskopische Verfahren Fourier-Transform Infrarot (FT-IR)-Spektroskopie, Nukleare Magnetresonanz (NMR)-Spektroskopie, Hochleistungsflüssigchromatographie (HPLC), Flüssigchromatographie-Massenspektrometrie (LC-MS) oder Gaschromatographie-Massenspektrometrie (GC-MS) ist.

8. Verfahren wie in einem der vorherigen Ansprüche beansprucht, wobei einer oder mehrere der Gehalte der N endogenen Metaboliten durch spektroskopische Techniken gemessen werden, die in Verbindung mit chemometrischen Verfahren eingesetzt werden, vorzugsweise wobei das chemometrische Verfahren Hauptkomponentenanalyse (PCA), Partial Least Square Projektionen auf latente Strukturen (PLS), Orthogonale PLS (OPLS), Diskriminante PLS-Analyse (PLS-DA) oder Orthogonale PLS-DA (OPLS-DA) ist.

9. Verfahren wie in einem der vorherigen Ansprüche beansprucht, wobei;
(a) eines oder mehrere der endogenen Stoffwechselprodukte werden durch Multi-Wellenlängen-Spektroskopie Messung gemessen; oder
(b) eines oder mehrere der endogenen Stoffwechselprodukte werden chemisch transformiert (d.h. derivatisiert) bevor sie gemessen werden.

10. Verfahren wie in einem der Ansprüche 1 bis 9 beansprucht, wobei eines oder mehrere der endogenen Stoffwechselprodukte unter Verwendung von Reagenzien gemessen werden, die individuell an die individuellen endogenen Metaboliten binden und welche daraufhin nachgewiesen werden.

11. Verfahren wie in einem der vorherigen Ansprüche beansprucht, wobei die Abnahmen der Gehalte der endogenen Stoffwechselprodukte (zwischen den Proben der Zielperson und den Kontrollproben oder den Proben, die in unterschiedlichen Zeitintervallen von der Zielperson entnommen wurden) signifikante Abnahmen sind.

12. N-(2-Chlor-3,4-dimethoxybenzylidenamino)guanidin, entweder als freie Base oder in Form des Salzes, zur Verwendung bei der Behandlung von rheumatoider Arthritis bei einer Zielperson, wobei es sich um eine Person handelt, bei der eine Störung des Stoffwechselprofils endogener Stoffwechselprodukte, die durch rheumatoide Arthritis hervorgerufen wird, durch ein Verfahren wie in einem der Ansprüche 1 bis 11 beansprucht nachgewiesen wurde.

## Revendications

1. Procédé *in vitro* de détection d'une perturbation du profil métabolique de métabolites endogènes chez un sujet, provoquée par la polyarthrite rhumatoïde (PR), le procédé comprenant les étapes consistant à :
(i) mesurer les niveaux de N métabolites endogènes dans un échantillon biologique prélevé sur le sujet, où N vaut 11, afin de produire un profil métabolique des N métabolites endogènes chez ce sujet ;
(ii) comparer les niveaux mesurés des N métabolites endogènes aux niveaux correspondants des métabolites endogènes dans un échantillon biologique prélevé sur un témoin ;
où les N métabolites endogènes sont :
EM1 Phénylalanine
EM2 Tyrosine
EM3 Isoleucine
EM8 Glycine
EM9 Glutamine
EM10 Méthionine
EM14 Lysine
EM15 Asparagine
EM16 Sérine
EM17 Tryptophane
EM18 Thréonine
où la perturbation du profil métabolique est une perturbation se traduisant par une diminution du niveau de chacun des N métabolites endogènes mesurés dans l'échantillon biologique prélevé sur le sujet comparativement aux niveaux correspondants de métabolites endogènes dans l'échantillon biologique prélevé sur le témoin ;
et où la perturbation du profil métabolique est due à la PR chez le sujet.

2. Procédé *in vitro* de détection d'une perturbation du profil métabolique de métabolites endogènes chez un sujet, provoquée par la polyarthrite rhumatoïde (PR), le procédé comprenant les étapes consistant à :
(i) mesurer les niveaux de N métabolites endogènes dans un échantillon biologique prélevé sur le sujet, où N vaut 11, afin de produire un profil métabolique des N métabolites endogènes chez ce sujet ;
(ii) comparer les niveaux mesurés des N métabolites endogènes aux niveaux correspondants des métabolites endogènes dans un échantillon biologique préalablement prélevé sur le sujet ;
où les N métabolites endogènes sont :
EM1 Phénylalanine
EM2 Tyrosine
EM3 Isoleucine
EM8 Glycine
EM9 Glutamine
EM10 Méthionine
EM14 Lysine
EM15 Asparagine
EM16 Sérine
EM17 Tryptophane
EM18 Thréonine
où la perturbation du profil métabolique est une perturbation se traduisant par une diminution du niveau de chacun des N métabolites endogènes mesurés dans l'échantillon biologique prélevé sur le sujet comparativement aux niveaux correspondants de métabolites endogènes dans l'échantillon biologique préalablement prélevé sur le sujet ;
et où la perturbation du profil métabolique est due à la PR chez le sujet.

3. Procédé de surveillance du taux de déclin ou du taux d'amélioration ou taux de rétablissement d'un profil métabolique sain chez un sujet, comprenant un procédé selon la revendication 2,
et comprenant en outre l'étape consistant à diviser la diminution des métabolites endogènes par l'intervalle de temps entre le prélèvement des premier (c'est-à-dire précédent) et deuxième échantillons sur le sujet.

4. Procédé selon l'une quelconque des revendications précédentes, dans lequel l'échantillon biologique est un fluide biologique, de préférence du sang ou du liquide synovial.

5. Procédé selon l'une quelconque des revendications précédentes, dans lequel le sujet est un animal à sang chaud.

6. Procédé selon la revendication 5, dans lequel le sujet est un mammifère, de préférence un humain.

7. Procédé selon l'une quelconque des revendications précédentes, dans lequel un ou plusieurs des niveaux des N métabolites endogènes sont mesurés par des techniques chromatographiques ou spectroscopiques, de préférence dans lequel la méthode chromatographique ou spectroscopique est la spectroscopie infrarouge à transformée de Fourier (IRTF), la spectroscopie par résonance magnétique nucléaire (RMN), la chromatographie liquide à haute performance (CLHP), la chromatographie en phase liquide couplée à la spectrométrie de masse (CPUSM) ou la chromatographie en phase gazeuse couplée à la spectrométrie de masse (CPG/SM).

8. Procédé selon l'une quelconque des revendications précédentes, dans lequel un ou plusieurs des niveaux des N métabolites endogènes sont mesurés par des techniques spectroscopiques utilisées conjointement à des méthodes chimiométriques, de préférence dans lequel la méthode chimiométrique est l'analyse en composantes principales (ACP), les projections par les moindres carrés partiels sur les structures latentes (PLS), la PLS orthogonale (OPLS), l'analyse discriminante PLS (PLS-DA) ou la PLS-DA orthogonale (OPLS-DA).

9. Procédé selon l'une quelconque des revendications précédentes, dans lequel :
(a) un ou plusieurs des métabolites endogènes sont mesurés en effectuant une mesure spectroscopique multi-longueur d'ondes ; ou
(b) un ou plusieurs des métabolites endogènes sont chimiquement transformés (c'est-à-dire dérivés) avant d'être mesurés.

10. Procédé selon l'une quelconque des revendications 1 à 9, dans lequel un ou plusieurs des métabolites endogènes sont mesurés en utilisant des réactifs qui se lient individuellement aux métabolites endogènes individuels, et qui sont ensuite détectés.

11. Procédé selon l'une quelconque des revendications précédentes, dans lequel les diminutions des niveaux des métabolites endogènes (entre le sujet et les échantillons témoins ou entre les échantillons prélevés à différentes intervalles de temps sur le sujet) sont des diminutions importantes.

12. N-(2-chloro-3,4-diméthoxybenzylidèneamino)guanidine, sous forme de base libre ou de sel, pour une utilisation dans le traitement de la polyarthrite rhumatoïde chez un sujet, le sujet étant un sujet chez qui une perturbation du profil métabolique des métabolites endogènes provoquée par la polyarthrite rhumatoïde a été détectée par un procédé selon l'une quelconque des revendications 1 à 11.
